# EUROPEAN PATENT APPLICATION

(11) **EP 4 144 817 A1**
(43) Date of publication of application: **08.03.2023**
(21) Application number: 21796332.1
(22) Date of filing: 16.04.2021
(51) Int. Cl.: C09K 11/06, C07D 487/04, H05B 33/10, H01L 51/50

(54) **ORGANIC ELECTROLUMINESCENCE ELEMENT MATERIAL AND ORGANIC ELECTROLUMINESCENCE ELEMENT**

(30) Priority: 30.04.2020 JP 2020080549
(71) Applicant: NIPPON STEEL Chemical & Material Co., Ltd., Tokyo 103-0027 (JP)
(72) Inventor: IKENAGA, Yuji, Tokyo 103-0027 (JP); KAI, Takahiro, Tokyo 103-0027 (JP); YOSHIDA, Kazunari, Tokyo 103-0027 (JP); HAYASHI, Kentaro, Tokyo 103-0027 (JP)
(74) Representative: Vossius & Partner Patentanwälte Rechtsanwälte mbB
(86) International application number: PCT/JP2021/015655
(87) International publication number: WO 2021/220838

(57) **Abstract**

To provide an organic EL device having high efficiency and extended lifetime while having a low driving voltage, and a material for an organic electroluminescent device suitable therefor. This material for an organic electroluminescent device is comprised of an indolocarbazole compound represented by the following general formula (1): 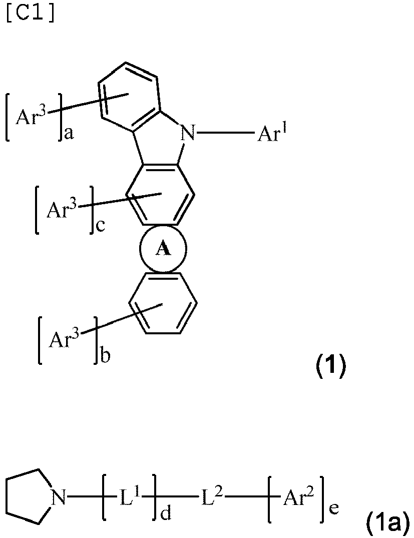 wherein a ring A is a heterocycle represented by formula (1a); Ar¹ and Ar² each represent an aromatic hydrocarbon group, an aromatic heterocyclic group, or a linked aromatic group; L² represents an aromatic heterocyclic group; Ar³ represents an aromatic hydrocarbon group or a linked aromatic group in which a plurality of these aromatic hydrocarbon groups are linked to each other; and a + b + c ≥ 1.

## Description

### Technical Field

The present invention relates to a material for an organic electroluminescent device and an organic electroluminescent device using the same.

### Background Art

Application of a voltage to an organic electroluminescent element or device (hereinafter, referred to as an organic EL device) allows injection of holes and electrons from an anode and a cathode, respectively, into a light-emitting layer. Then, in the light-emitting layer, injected holes and electrons recombine to generate excitons. At this time, according to statistical rules of electron spins, singlet excitons and triplet excitons are generated at a ratio of 1:3. Regarding a fluorescence-emitting organic EL device using light emission from singlet excitons, it is said that the internal quantum efficiency thereof has a limit of 25%. Meanwhile, regarding a phosphorescent organic EL device using light emission from triplet excitons, it is known that intersystem crossing is efficiently performed from singlet excitons, the internal quantum efficiency is enhanced to 100%.

However, extending the lifetime of a phosphorescent organic EL device has been a technical challenge.

Further, highly efficient organic EL devices utilizing delayed fluorescence have been developed recently. For example, Patent Literature 1 discloses an organic EL device utilizing a TTF (Triplet-Triplet Fusion) mechanism, which is one of delayed fluorescence mechanisms. The TTF mechanism utilizes a phenomenon in which singlet excitons are generated due to collision of two triplet excitons, and it is thought that the internal quantum efficiency can be theoretically raised to 40%. However, since the efficiency is lower compared to phosphorescent organic EL devices, further improvement in efficiency is required.

Meanwhile, patent Literature 2 discloses an organic EL device utilizing a TADF (Thermally Activated Delayed Fluorescence) mechanism. The TADF mechanism utilizes a phenomenon in which reverse intersystem crossing from triplet excitons to singlet excitons is generated in a material having a small energy difference between a singlet level and a triplet level, and it is thought that the internal quantum efficiency can be theoretically raised to 100%. However, further improvement in lifetime characteristics is required as in the case of the phosphorescent device.

### Citation List

### Patent Literature

Patent Literature 1: WO2010/134350 A
Patent Literature 2: WO2011/070963 A
Patent Literature 3: WO2008/056746 A
Patent Literature 4: Japanese Patent Laid-Open No. 2003-133075
Patent Literature 5: WO2013/062075 A
Patent Literature 6: US2014/0374728 A
Patent Literature 7: US2014/0197386 A
Patent Literature 8: US2015/0001488 A
Patent Literature 9: WO2011/136755 A
Patent Literature 10: KR2013/132226 A
Patent Literature 11: WO2016/042997A

Patent Literatures 3 and 10 disclose use of an indolocarbazole compound as a host material. Patent Literature 4 discloses use of a biscarbazole compound as a host material.

Patent Literatures 5 and 6 disclose use of a biscarbazole compound as a mixed host. Patent Literatures 7 and 8 disclose use of an indolocarbazole compound and a biscarbazole compound as a mixed host.

Patent Literature 9 discloses use of a host material in which a plurality of hosts containing an indolocarbazole compound is premixed.

Patent Literature 11 discloses use of a host material in which a plurality of indolocarbazole compounds is premixed.

However, none of these can be said to be sufficient, and further improvements are desired.

### Summary of Invention

In order to apply organic EL devices to display devices such as flat panel displays, it is necessary to improve the luminous efficiency of devices and at the same time, to sufficiently secure the lifetime characteristics of the devices. In view of the above circumstances, an object of the present invention is to provide a practically useful organic EL device having high efficiency and extended lifetime while having a low driving voltage, and a compound suitable therefor.

As a result of intensive studies, the present inventors have found that an organic EL device exhibits excellent characteristics by using a fused aromatic heterocycle compound represented by the following general formula (1), and have completed the present invention.

The present invention is a material for an organic EL device comprising a compound represented by the following general formula (1).

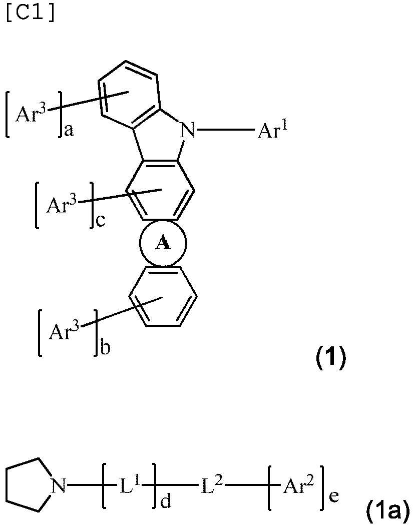

In the formula, a ring A is a heterocycle represented by formula (1a), and the ring A is fused to an adjacent ring at any position; each Ar¹ independently represents a substituted or unsubstituted aromatic hydrocarbon group having 6 to 30 carbon atoms, a substituted or unsubstituted aromatic heterocyclic group having 3 to 11 carbon atoms, or a substituted or unsubstituted linked aromatic group in which two to five of these aromatic rings are linked to each other; each Ar² independently represents a substituted or unsubstituted aromatic hydrocarbon group having 6 to 30 carbon atoms, a substituted or unsubstituted aromatic heterocyclic group having 3 to 17 carbon atoms, or a substituted or unsubstituted linked aromatic group in which two to five of these aromatic rings are linked to each other; L¹ independently represents a substituted or unsubstituted aromatic hydrocarbon group having 6 to 30 carbon atoms, or a substituted or unsubstituted aromatic heterocyclic group having 3 to 11 carbon atoms; L² represents a substituted or unsubstituted aromatic heterocyclic group having 3 to 11 carbon atoms; Ar³ independently represents a substituted or unsubstituted aromatic hydrocarbon group having 6 to 30 carbon atoms, or a substituted or unsubstituted linked aromatic group in which two to five of these aromatic hydrocarbon groups are linked to each other; and a and b each independently represent an integer of 0 to 4, and c represents an integer of 0 to 2. However, a + b + c ≥ 1. d independently represents the number of repetitions and an integer of 0 to 3, and e represents the number of substitutions and an integer of 0 to 5.

The compound represented by the general formula (1) may have L² as a nitrogen-containing aromatic group having 3 to 5 carbon atoms. Ar¹, Ar², and L¹ each can also be a substituted or unsubstituted aromatic hydrocarbon group having 6 to 24 carbon atoms, or a substituted or unsubstituted linked aromatic group in which two to five of the aromatic hydrocarbon groups are linked to each other, and preferably d = 0.

The compound represented by the general formula (1) is preferably a compound represented by any of the following formulas (2) to (7): 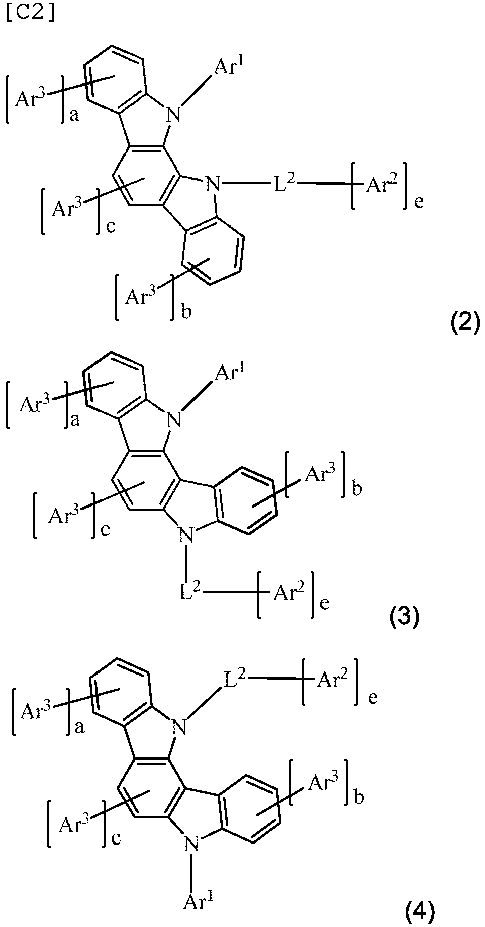 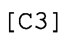 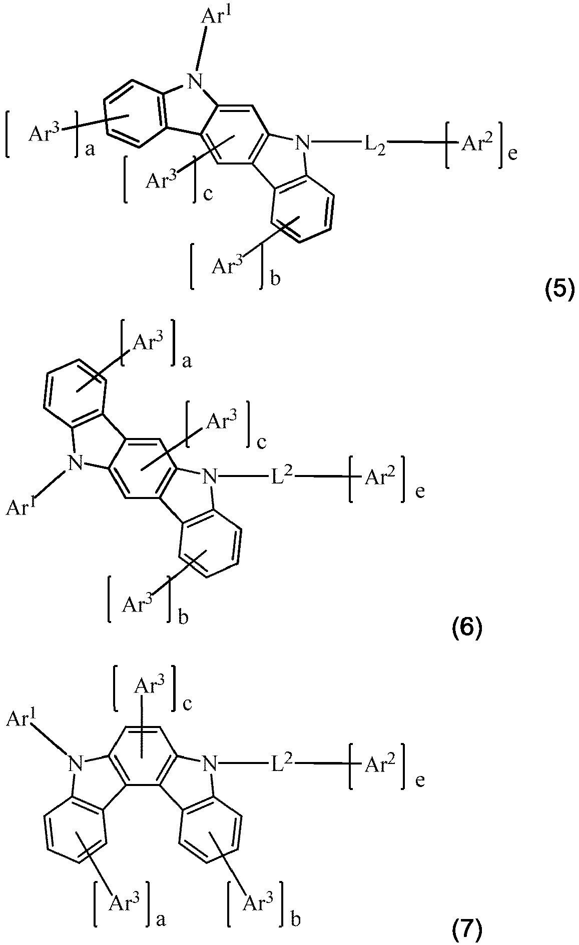 wherein L², Ar¹, Ar², Ar³, a, b, c, and e are as defined for the general formula (1).

In the general formula (1) or formulas (2) to (7), it is preferable that at least one selected from a and b be 0, and c ≥ 1.

The present invention is an organic EL device having a plurality of organic layers between an anode and a cathode, wherein at least one layer of the organic layers is an organic layer comprising the material for an organic EL device.

The organic layer comprising the material for an organic EL device may include a material for an organic EL device and at least one of compounds represented by the general formulas (8) to (10): 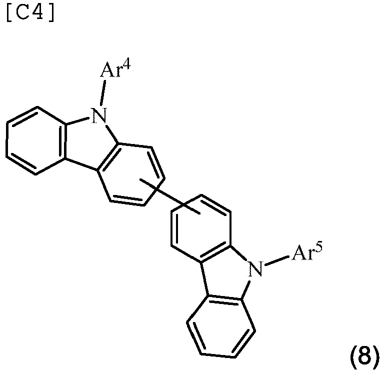 wherein Ar⁴ and Ar⁵ each independently represent a substituted or unsubstituted aromatic hydrocarbon group having 6 to 30 carbon atoms, a substituted or unsubstituted aromatic heterocyclic group having 3 to 17 carbon atoms, or a substituted or unsubstituted linked aromatic group in which two to five of these aromatic rings are linked to each other.

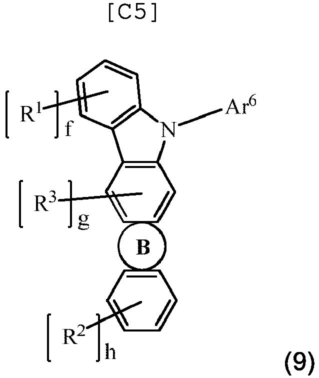

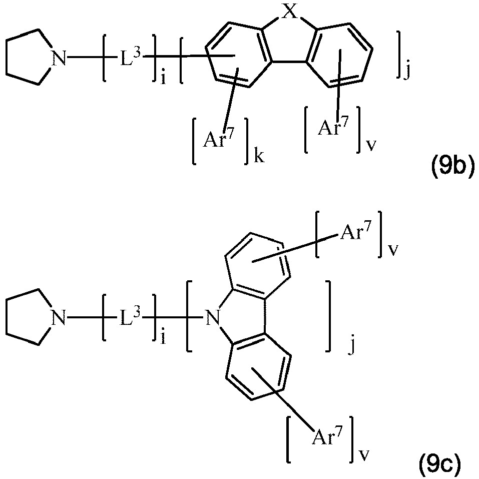

In the formula, a ring B is a heterocycle represented by formula (9b) or (9c), and the ring B is fused to an adjacent ring at any position; L³ independently represents a substituted or unsubstituted aromatic hydrocarbon group having 6 to 30 carbon atoms; and X represents NAr⁸, O, or S.

Ar⁶, Ar⁷, and Ar⁸ each independently represent a substituted or unsubstituted aromatic hydrocarbon group having 6 to 30 carbon atoms, a substituted or unsubstituted aromatic heterocyclic group having 3 to 17 carbon atoms, or a substituted or unsubstituted linked aromatic group in which two to five of these aromatic rings are linked to each other. i and j each independently represent an integer of 0 to 3; k and v represent the number of substitutions; and k represents an integer of 0 to 3, and v independently represents an integer of 0 to 4. However, i + j is an integer of 1 or more.

R¹ to R³ each independently represent a cyano group, an alkyl group having 1 to 20 carbon atoms, an aralkyl group having 7 to 38 carbon atoms, an alkenyl group having 2 to 20 carbon atoms, an alkynyl group having 2 to 20 carbon atoms, a dialkylamino group having 2 to 40 carbon atoms, a diarylamino group having 12 to 44 carbon atoms, a diaralkylamino group having 14 to 76 carbon atoms, an acyl group having 2 to 20 carbon atoms, an acyloxy group having 2 to 20 carbon atoms, an alkoxy group having 1 to 20 carbon atoms, an alkoxycarbonyl group having 2 to 20 carbon atoms, an alkoxycarbonyloxy group having 2 to 20 carbon atoms, an alkylsulfonyl group having 1 to 20 carbon atoms, a substituted or unsubstituted aromatic hydrocarbon group having 6 to 30 carbon atoms, or a substituted or unsubstituted aromatic heterocyclic group having 3 to 17 carbon atoms; and each may have a substituent.

f and h each independently represent an integer of 0 to 4, and g represents an integer of 0 to 2.

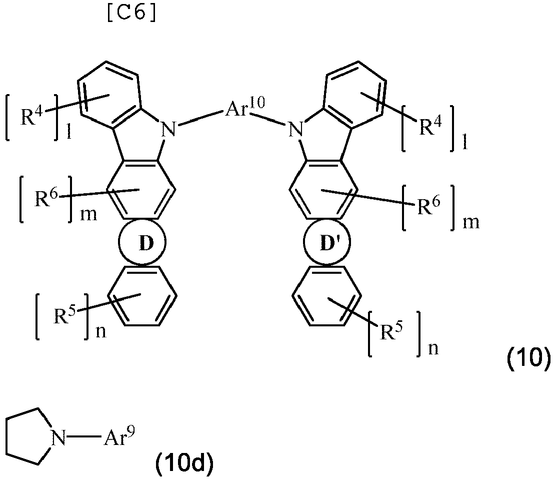

In the formula, rings D and D' are each a heterocycle represented by formula (10d), and the rings D and D' are each independently fused to an adjacent ring at any position and Ar⁹ independently has the same meaning as Ar⁶ in the general formula (9); R⁴ to R⁶ each independently have the same meaning as R¹ to R³; and l and n each independently represent an integer of 0 to 4, and m independently represents an integer of 0 to 2. Ar¹⁰ represents a substituted or unsubstituted aromatic hydrocarbon group having 6 to 30 carbon atoms, a substituted or unsubstituted aromatic heterocyclic group having 3 to 17 carbon atoms, or a substituted or unsubstituted linked aromatic group in which two to five of them are linked to each other.

The organic layer can be at least one layer selected from the group comprising of a light-emitting layer, a hole injection layer, a hole transport layer, an electron transport layer, an electron injection layer, a hole blocking layer, and an electron blocking layer, and is preferably a light-emitting layer. This light-emitting layer contains at least one light-emitting dopant.

In addition, the present invention is a mixed composition comprising a compound represented by the general formula (1) and a compound represented by the general formula (8), (9), or (10). It is desirable that a difference in 50% weight reduction temperatures of these compounds is within 20°C.

The present invention is also a method for producing an organic EL device, comprising producing a light-emitting layer by use of the mixed composition.

Since the material for an organic EL device of the present invention has an aromatic heterocyclic group on the fused aromatic heterocyclic ring, it is assumed that designing the number of substituents and the linking scheme as appropriate allowed a high-level control of hole injection transport properties of the material.

In addition, changing the kind of substituents binding to L² and the position of substituent introduction allowed a high-level control of electron injection transport properties of the material.

Given the above characteristics, it is considered that the material of the present invention is a material having both charge (electron and hole) injection transport properties suitable for device configuration, and unexpected characteristics such as reduction in driving voltage and high luminous efficiency of the device could be achieved by using this material in the organic EL device.

It is also assumed that the material for an organic EL device exhibited good amorphous properties and high thermal stability and was extremely stable in the excited state, and therefore, it is thought that the organic EL device using this material had an unexpectedly extended lifetime and exhibited a practical level of durability.

### Brief Description of Drawing

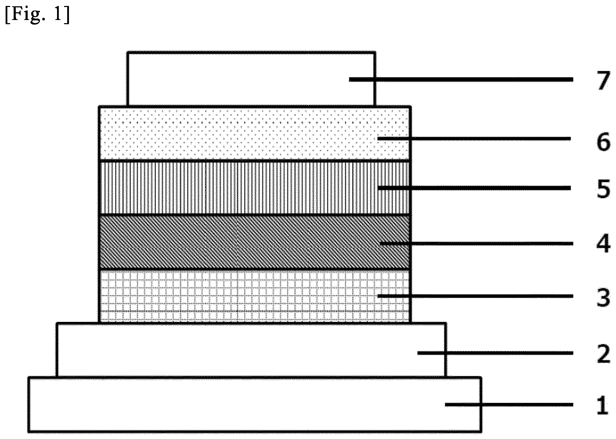

[Figure 1] Figure 1 is a cross-sectional view showing one structure example of an organic EL device.

### Description of Embodiments

A material for an organic EL device of the present invention is represented by the general formula (1). In the general formula (1), a ring A is a heterocycle represented by formula (1a), and the ring A is fused to an adjacent ring at any position.

Ar¹ independently represents a substituted or unsubstituted aromatic hydrocarbon group having 6 to 30 carbon atoms, a substituted or unsubstituted aromatic heterocyclic group having 3 to 11 carbon atoms, or a substituted or unsubstituted linked aromatic group in which two to five of these aromatic rings are linked to each other, preferably a substituted or unsubstituted aromatic hydrocarbon group having 6 to 24 carbon atoms, or a substituted or unsubstituted linked aromatic group in which two to five of these aromatic hydrocarbon groups are linked to each, and more preferably a substituted or unsubstituted aromatic hydrocarbon group having 6 to 18 carbon atoms, or a substituted or unsubstituted linked aromatic group in which two to five of these aromatic hydrocarbon groups are linked to each other.

Ar² independently represents a substituted or unsubstituted aromatic hydrocarbon group having 6 to 30 carbon atoms, a substituted or unsubstituted aromatic heterocyclic group having 3 to 17 carbon atoms, or a substituted or unsubstituted linked aromatic group in which two to five of these aromatic rings are linked to each other, preferably a substituted or unsubstituted aromatic hydrocarbon group having 6 to 24 carbon atoms, or a substituted or unsubstituted linked aromatic group in which two to five of the aromatic hydrocarbon groups are linked to each other, and more preferably a substituted or unsubstituted aromatic hydrocarbon group having 6 to 18 carbon atoms, or a substituted or unsubstituted linked aromatic group in which two to five of these aromatic hydrocarbon groups are linked to each other.

In the case of Ar¹ and Ar² being an unsubstituted aromatic hydrocarbon group, aromatic heterocyclic group, or linked aromatic group, specific examples thereof include a group generated by removing one hydrogen from benzene, naphthalene, acenaphthene, acenaphthylene, azulene, anthracene, chrysene, pyrene, phenanthrene, triphenylene, fluorene, benzo[a]anthracene, tetracene, pentacene, hexacene, coronene, heptacene, pyridine, pyrimidine, triazine, thiophene, isothiazole, thiazole, pyridazine, pyrrole, pyrazole, imidazole, triazole, thiadiazole, pyrazine, furan, isoxazole, quinoline, isoquinoline, quinoxaline, quinazoline, thiadiazole, phthalazine, tetrazole, indole, benzofuran, benzothiophene, benzoxazole, benzothiazole, indazole, benzimidazole, benzotriazole, benzisothiazole, benzothiadiazole, purine, pyranone, coumarin, isocoumarin, chromone, dibenzofuran, dibenzothiophene, dibenzoselenophene, carbazole, or compounds in which two to five of these are linked to each other. Preferred examples thereof include a group generated by benzene, naphthalene, acenaphthene, acenaphthylene, azulene, anthracene, chrysene, pyrene, phenanthrene, triphenylene, fluorene, benzo[a]anthracene, tetracene, pentacene, hexacene, coronene, heptacene, or compounds in which two to five of these are linked to each other. More preferred examples thereof include benzene, naphthalene, acenaphthene, acenaphthylene, azulene, anthracene, chrysene, pyrene, phenanthrene, triphenylene, fluorene, benzo[a]anthracene, tetracene, or compounds in which two to five of these are linked to each other. Further preferred is a phenyl group, a biphenyl group, or a terphenyl group. The terphenyl group may be linked linearly or branched.

In the case of Ar¹ being an aromatic heterocyclic group, it is limited to an aromatic heterocyclic group having 3 to 11 carbon atom. Hence, dibenzofuran, dibenzothiophene, dibenzoselenophene, and carbazole are excluded from the above. Preferable examples of the unsubstituted aromatic heterocyclic group include a group generated from pyridine, pyrimidine, triazine, thiophene, isothiazole, thiazole, pyridazine, pyrrole, pyrazole, imidazole, triazole, thiadiazole, pyrazine, furan, isoxazole, and oxazole. More preferred is a group generated from pyridine, pyrimidine, or triazine.

L² represents a substituted or unsubstituted aromatic heterocyclic group having 3 to 11 carbon atoms, preferably a substituted or unsubstituted aromatic heterocyclic group having 3 to 5 carbon atoms, and more preferably a substituted or unsubstituted nitrogen-containing aromatic group having 3 to 5 carbon atoms.

In the case of L² being an unsubstituted aromatic heterocyclic group, specific examples thereof include a group generated from pyridine, pyrimidine, triazine, thiophene, isothiazole, thiazole, pyridazine, pyrrole, pyrazole, imidazole, pyrazine, furan, isoxazole, oxazole, quinoline, isoquinoline, quinoxaline, quinazoline, benzotriazine, phthalazine, indole, benzofuran, benzothiophene, benzoxazole, benzothiazole, indazole, benzimidazole, benzisothiazole, or benzothiadiazole. Preferred examples include a group generated from pyridine, pyrimidine, triazine, thiophene, isothiazole, thiazole, pyridazine, pyrrole, pyrazole, imidazole, triazole, thiadiazole, pyrazine, furan, isoxazole, and oxazole. More preferred is a group generated from pyridine, pyrimidine, or triazine.

L¹ represents a substituted or unsubstituted aromatic hydrocarbon group having 6 to 30 carbon atoms, or a substituted or unsubstituted aromatic heterocyclic group having 3 to 11 carbon atoms, preferably a substituted or unsubstituted aromatic hydrocarbon group having 6 to 24 carbon atoms, more preferably a substituted or unsubstituted aromatic hydrocarbon group having 6 to 18 carbon atoms. In the case of L¹ being an unsubstituted aromatic hydrocarbon group, specific examples thereof are the same as in the case of Ar¹ and Ar², and in the case of being an unsubstituted aromatic heterocyclic group, specific examples thereof are the same as in the case of L2. Note that L¹ is a divalent group, and L² is a group having a valency of e+1.

Ar³ represents a substituted or unsubstituted aromatic hydrocarbon group having 6 to 30 carbon atoms, or a substituted or unsubstituted linked aromatic group in which two to five of these aromatic hydrocarbon groups are linked to each other, and preferred is a substituted or unsubstituted aromatic hydrocarbon group having 6 to 24 carbon atoms, or a substituted or unsubstituted linked aromatic group in which two to five of the aromatic hydrocarbon groups are linked to each other and more preferred is a substituted or unsubstituted aromatic hydrocarbon group having 6 to 18 carbon atoms, or a substituted or unsubstituted linked aromatic group in which two to five of these aromatic hydrocarbon groups are linked to each other. In the case of Ar³ being an unsubstituted aromatic hydrocarbon group, specific examples thereof are the same as in the case of Ar¹ and Ar². Further preferred is a phenyl group, a biphenyl group, or a terphenyl group. The terphenyl group may be linked linearly or branched.

a and b each independently represent an integer of 0 to 4, and c represents an integer of 0 to 2. However, a + b + c ≥ 1. Preferably, a and b each independently represent an integer of 0 to 1, and c may be 1 or 2. Further preferably, at least one selected from a and b is 0.
d represents the number of repetitions and is an integer of 0 to 3, preferably an integer of 0 to 1, and more preferably 0. e represents the number of substitutions and an integer of 0 to 5, and is preferably 0 to 3 and more preferably 0 to 2.

In the present specification, an unsubstituted aromatic hydrocarbon group, aromatic heterocyclic group, or linked aromatic group may each have a substituent. In the case of having a substituent, the substituent is preferably deuterium, halogen, a cyano group, a triarylsilyl group, an aliphatic hydrocarbon group having 1 to 10 carbon atoms, an alkenyl group having 2 to 5 carbon atoms, an alkoxy group having 1 to 5 carbon atoms, or a diarylamino group having 12 to 44 carbon atoms. In the case of the aliphatic hydrocarbon group having 1 to 10 carbon atoms, the substituent may be linear, branched, or cyclic.

Note that the number of substituents is 0 to 5 and preferably 0 to 2. When an aromatic hydrocarbon group and an aromatic heterocyclic group have substituents, the calculation of the number of carbon atoms does not include the number of carbon atoms of the substituents. However, it is preferred that the total number of carbon atoms including the number of carbon atoms of substituents satisfy the above range.

Specific examples of the substituents include cyano, methyl, ethyl, propyl, i-propyl, butyl, t-butyl, pentyl, cyclopentyl, hexyl, cyclohexyl, heptyl, octyl, nonyl, decyl, vinyl, propenyl, butenyl, pentenyl, methoxy, ethoxy, propoxy, butoxy, pentoxy, diphenylamino, naphthylphenylamino, dinaphthylamino, dianthranylamino, diphenanthrenylamino, and dipyrenylamino. Preferred examples thereof include cyano, methyl, ethyl, propyl, butyl, pentyl, hexyl, heptyl, octyl, diphenylamino, naphthylphenylamino, or dinaphthylamino.

In the present specification, the linked aromatic group refers to an aromatic group in which the carbon atoms of the aromatic rings in the aromatic group are linked to each other by a single bond. It is an aromatic group in which two or more aromatic groups are linked to each other, and they may be linear or branched. The aromatic group may be an aromatic hydrocarbon group or an aromatic heterocyclic group, and the plurality of aromatic groups may be the same or different. The aromatic group corresponding to the linked aromatic group is different from the substituted aromatic group.

In the present specification, it is understood that hydrogen may be deuterium. In other words, for example, in the general formula (1) to (10), some or all of the H may be possessed by the indolocarbazole-like skeleton, and substituents such as R¹ and Ar¹ may be deuterium.

Preferred aspects of the compounds represented by the general formula (1) include compounds represented by any of the general formulas (2) to (7) and more preferably compounds represented by any of formulas (2) to (5). In the formulas (2) to (7), the same symbols as in the general formula (1) have the same meaning.

Specific examples of the compounds represented by the general formula (1) are shown below, but are not limited to these exemplified compounds.

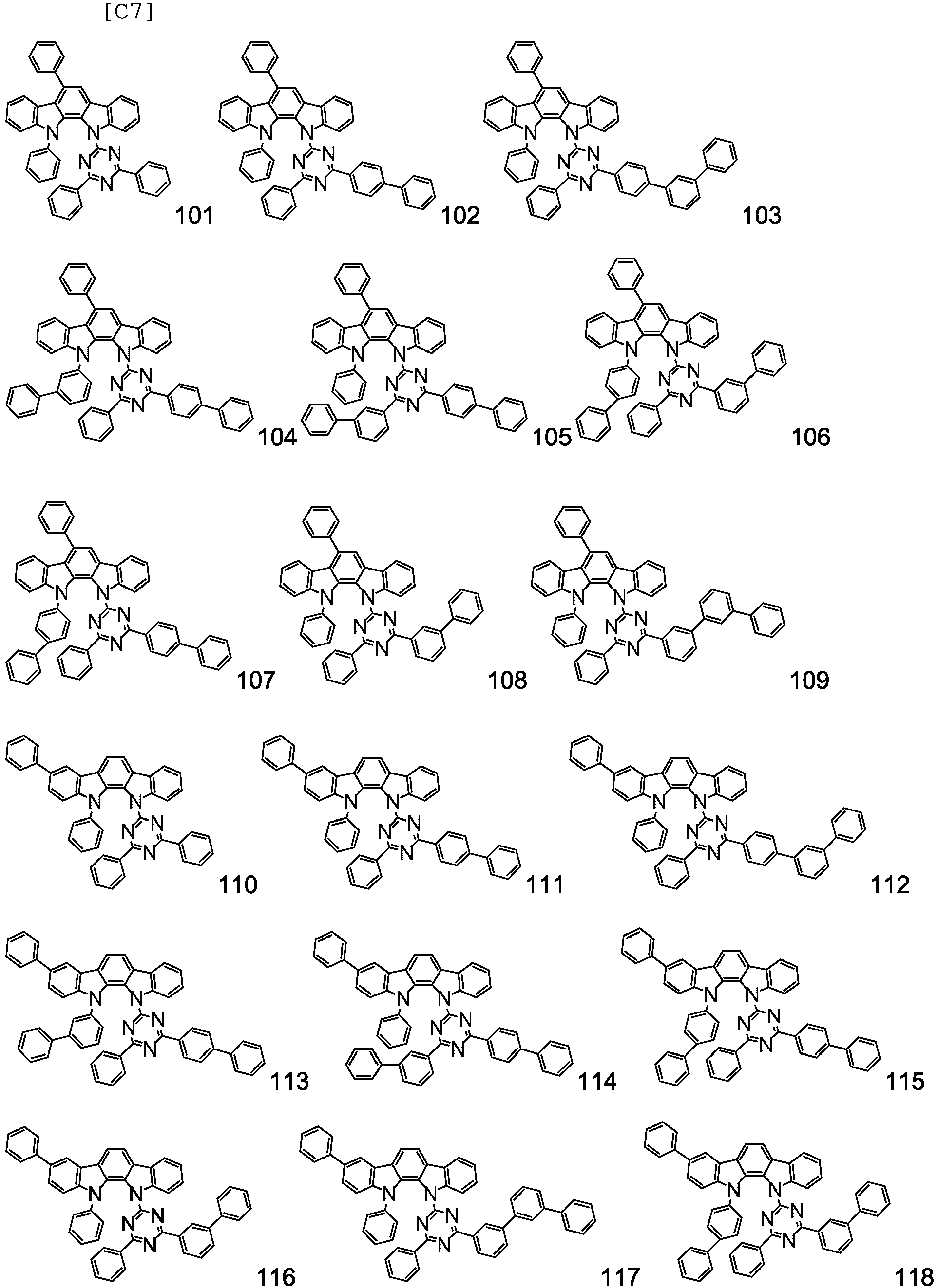

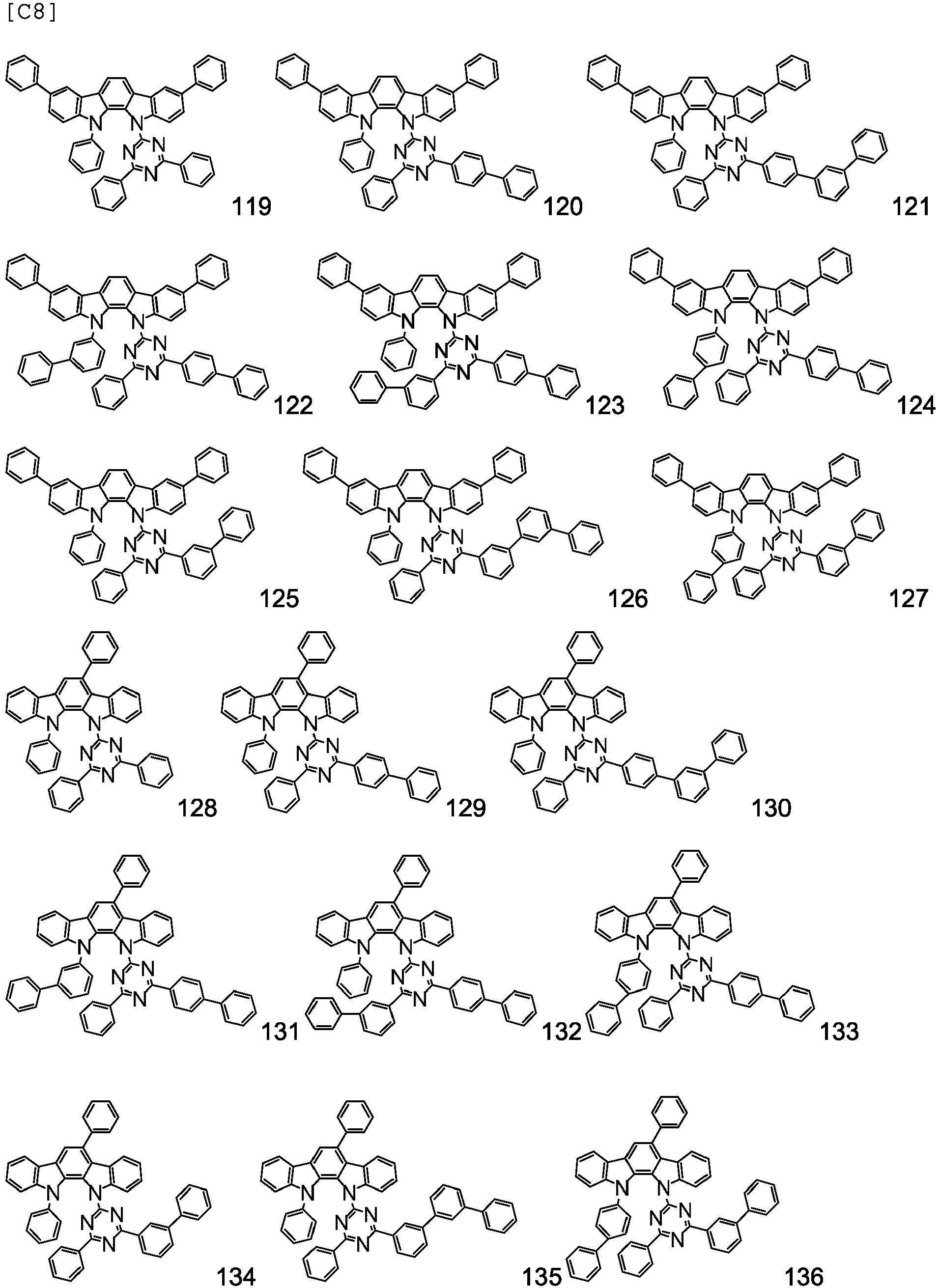

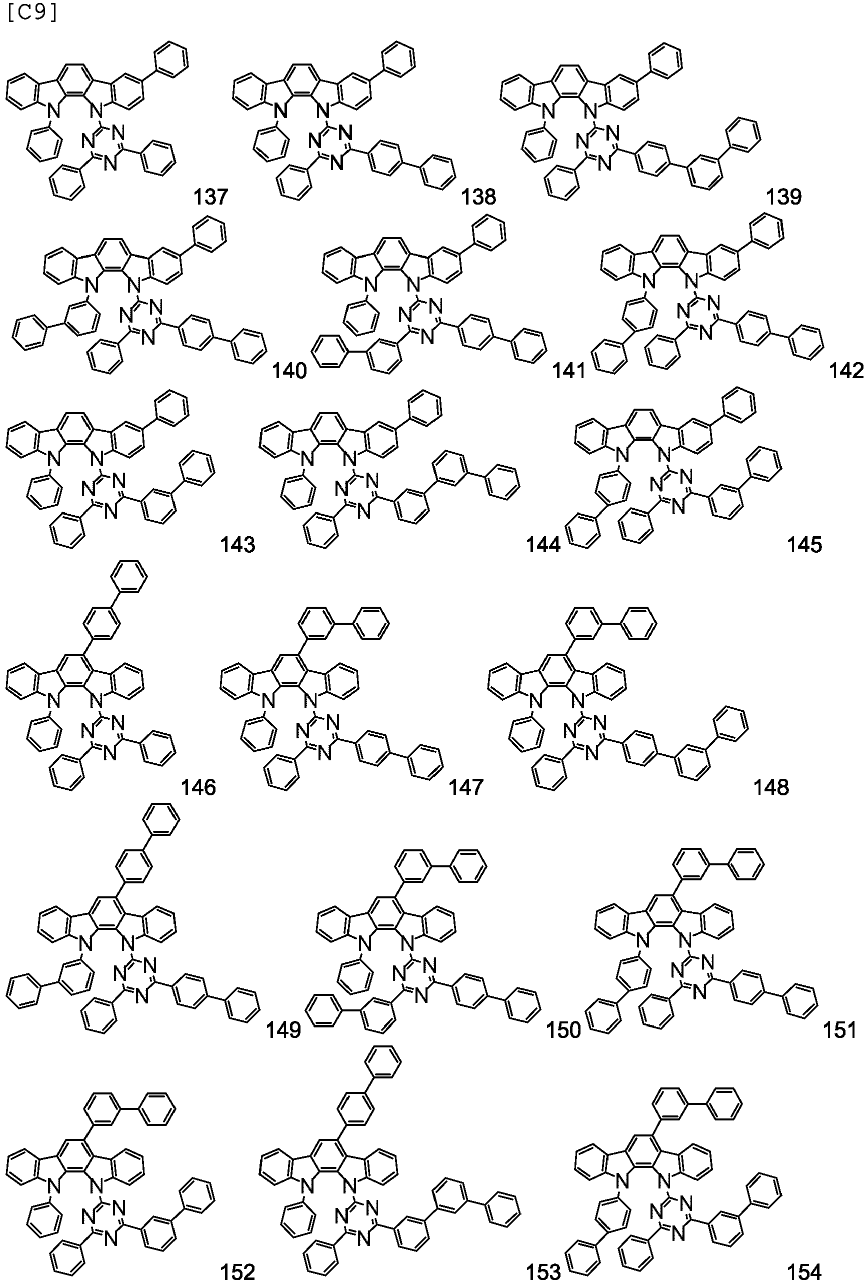

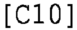

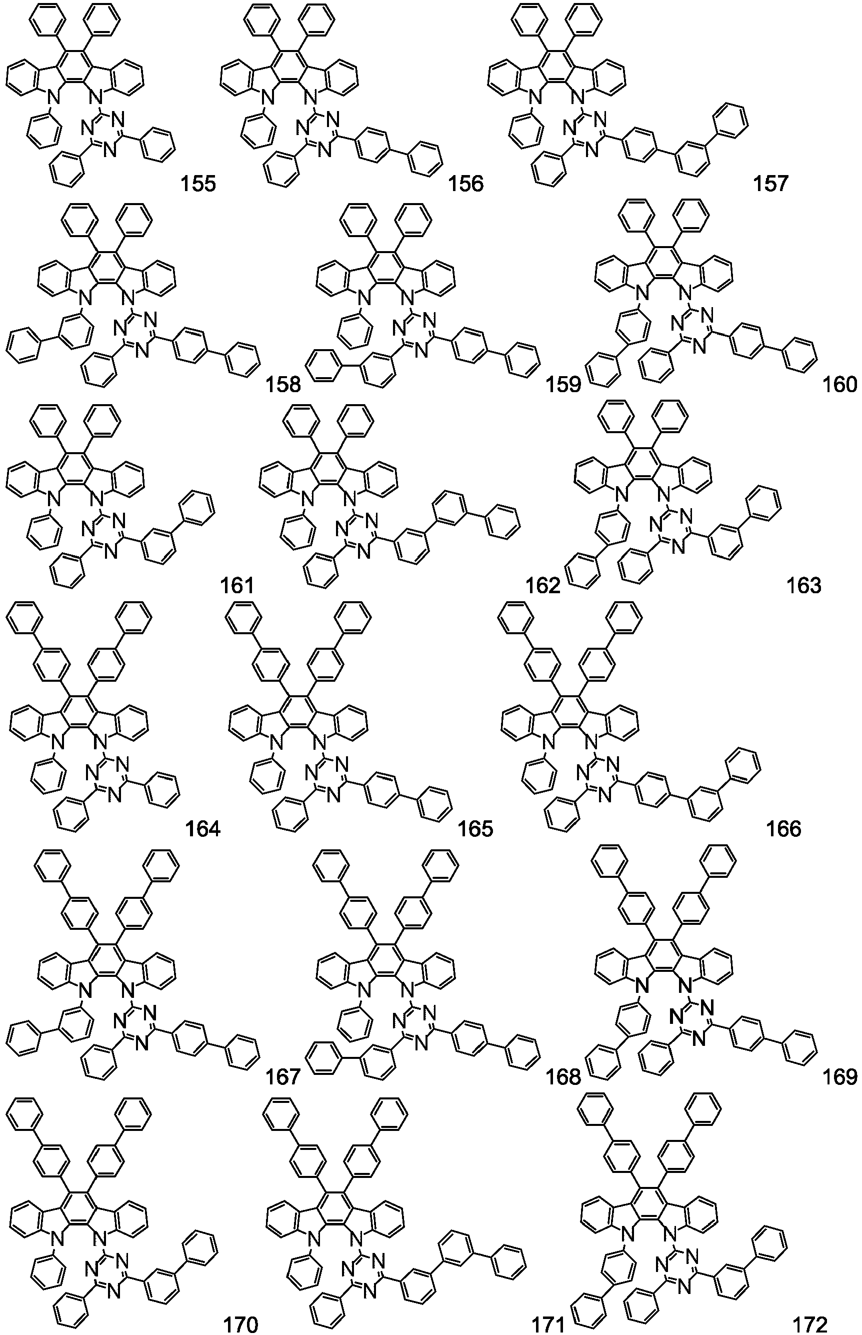

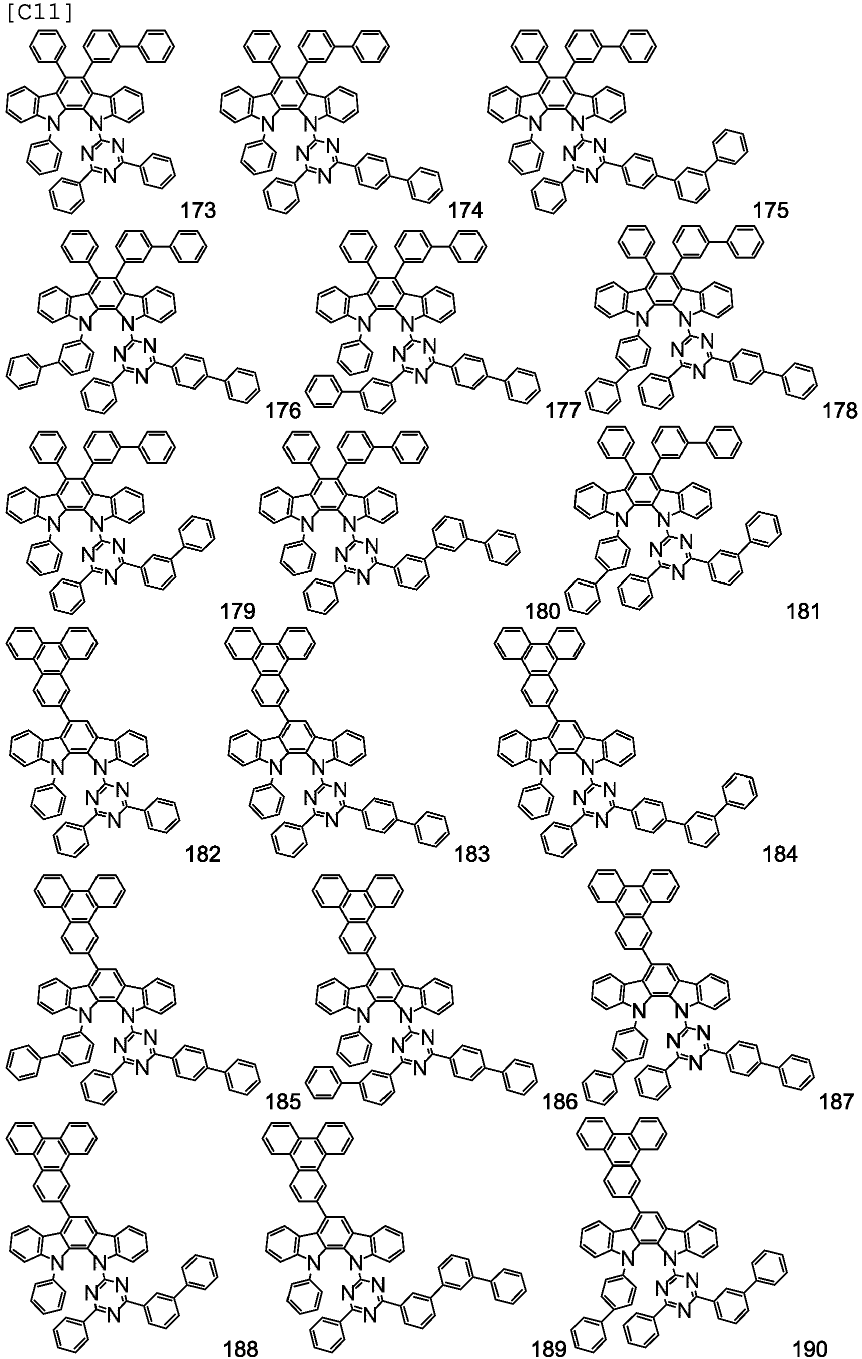

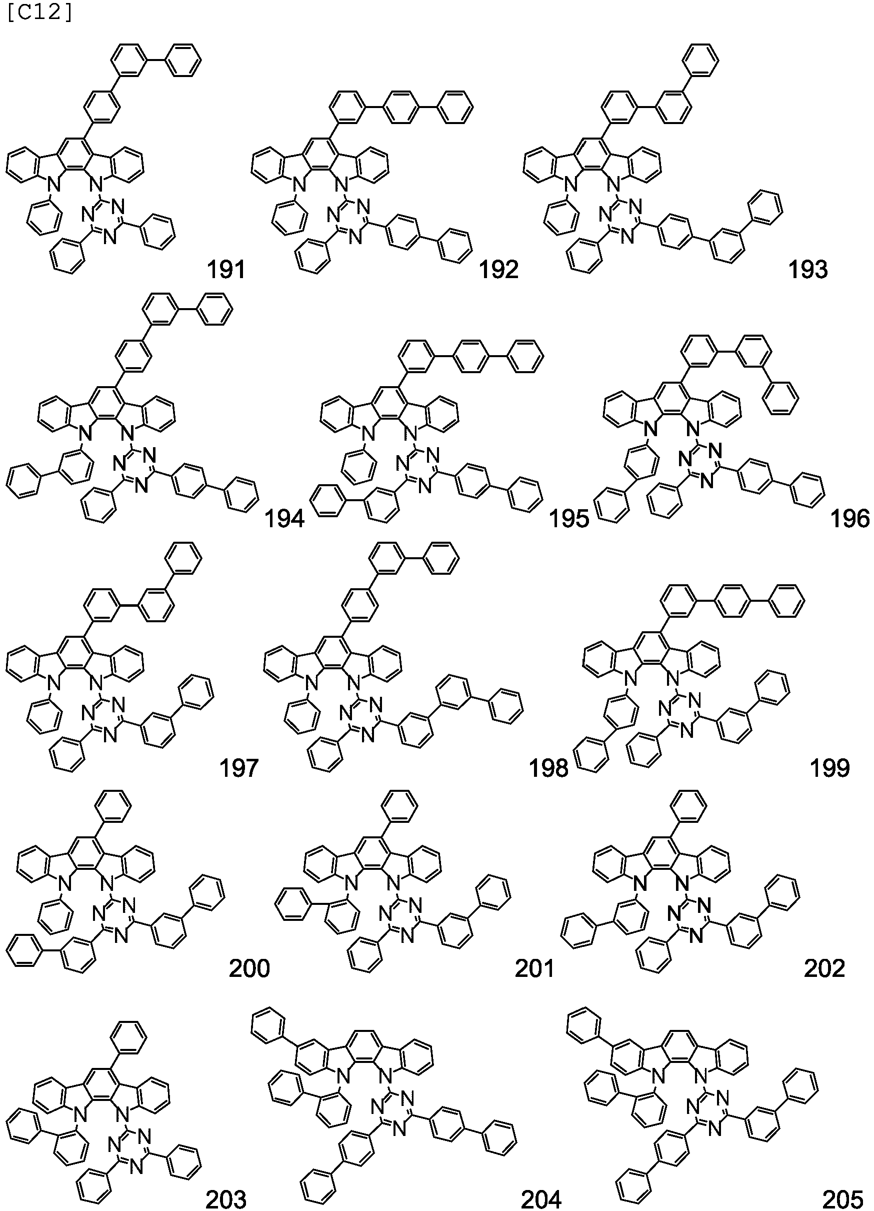

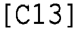

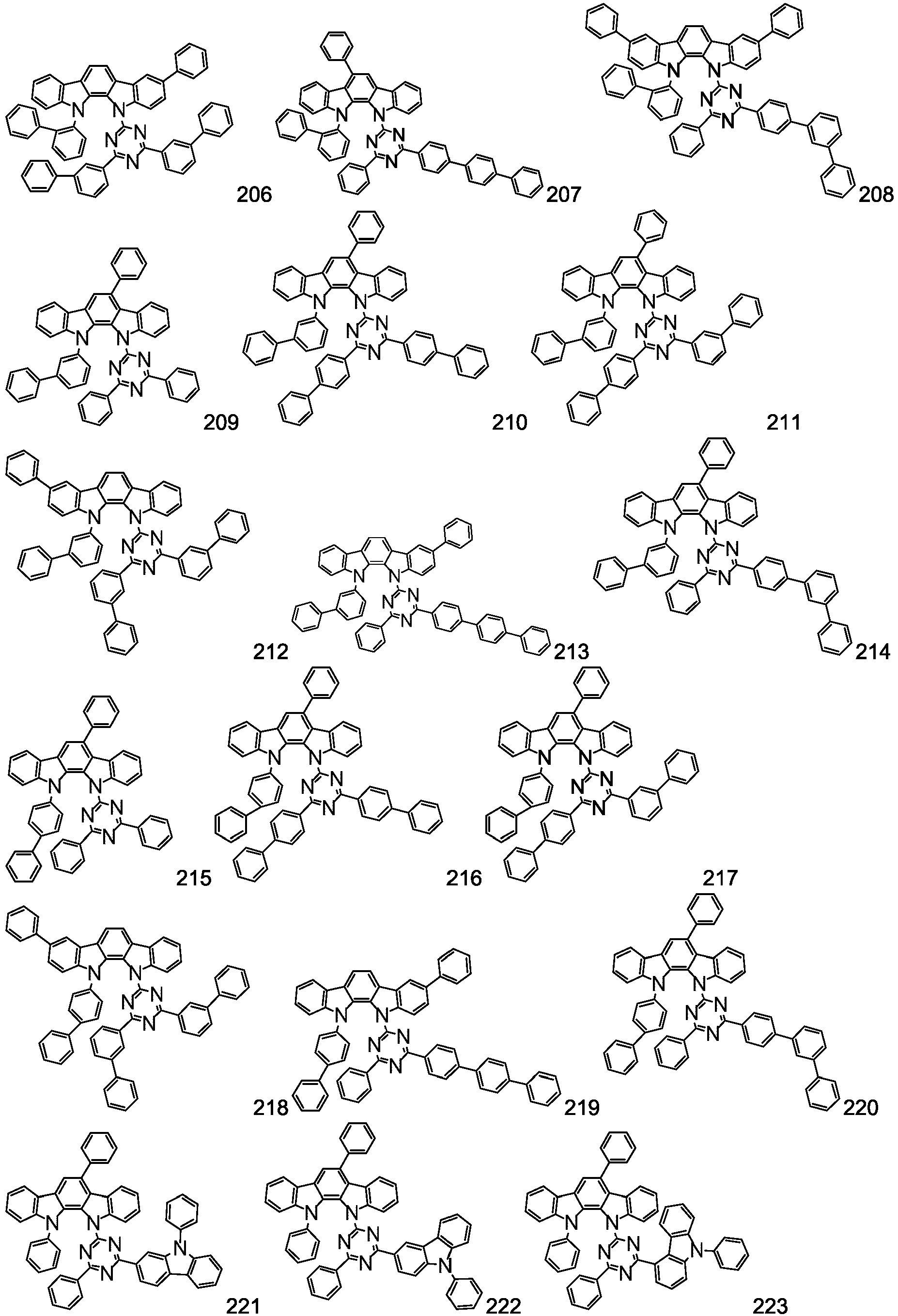

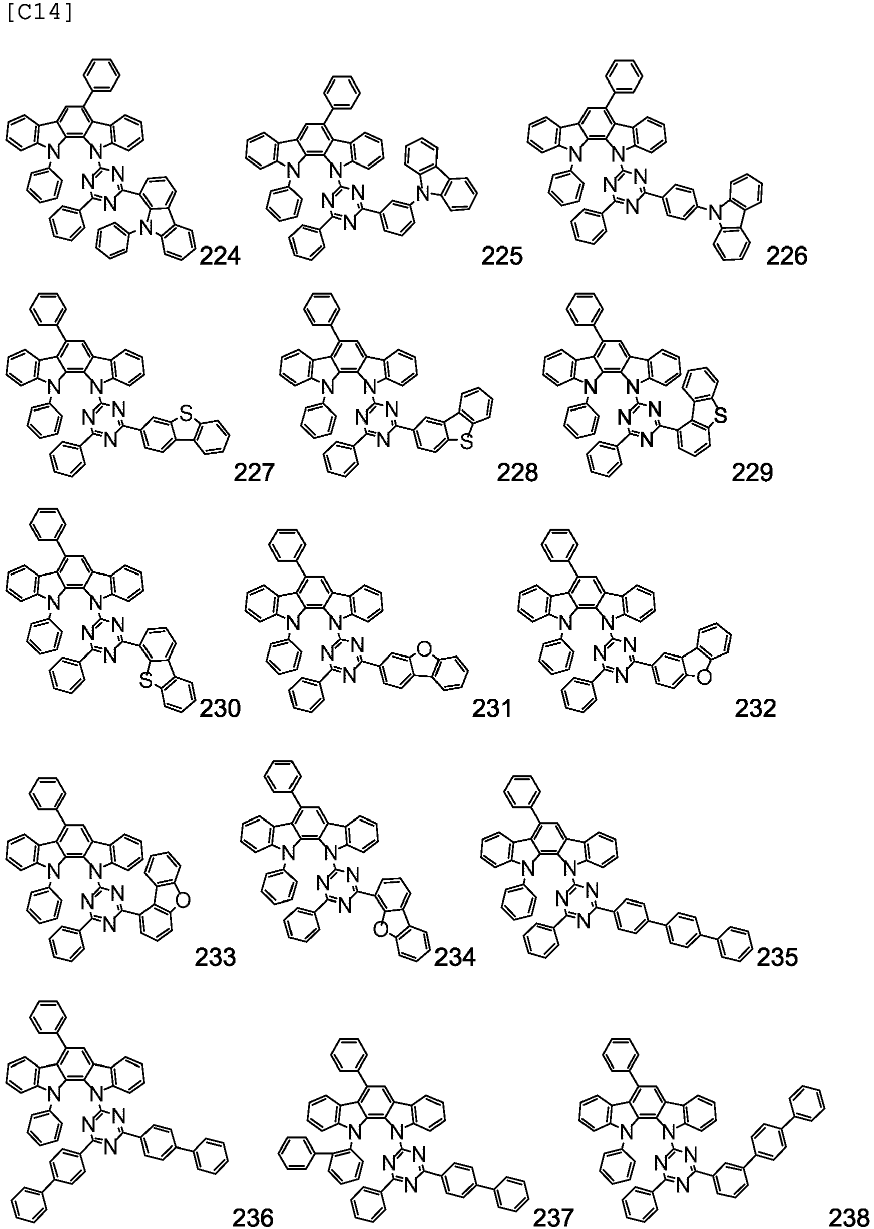

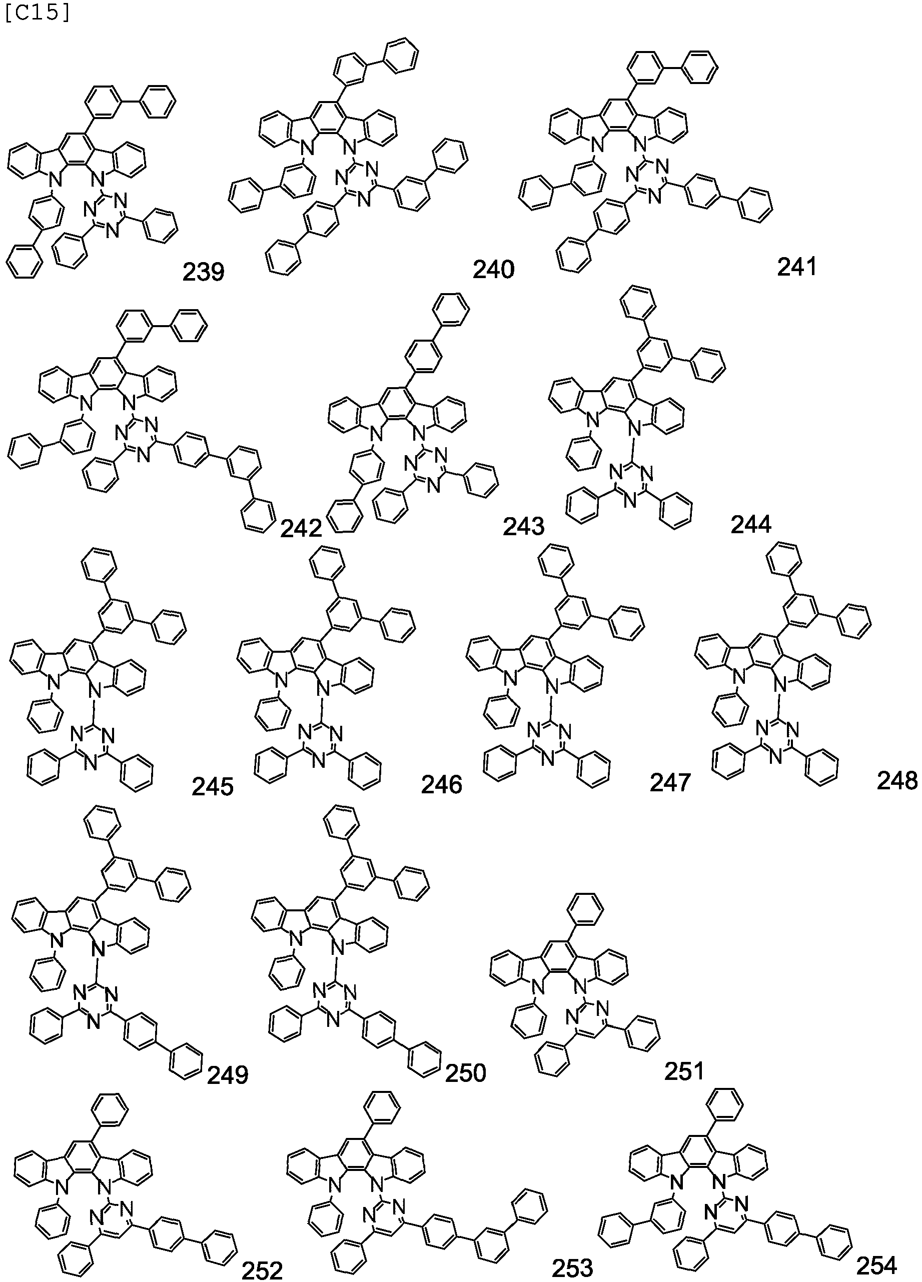

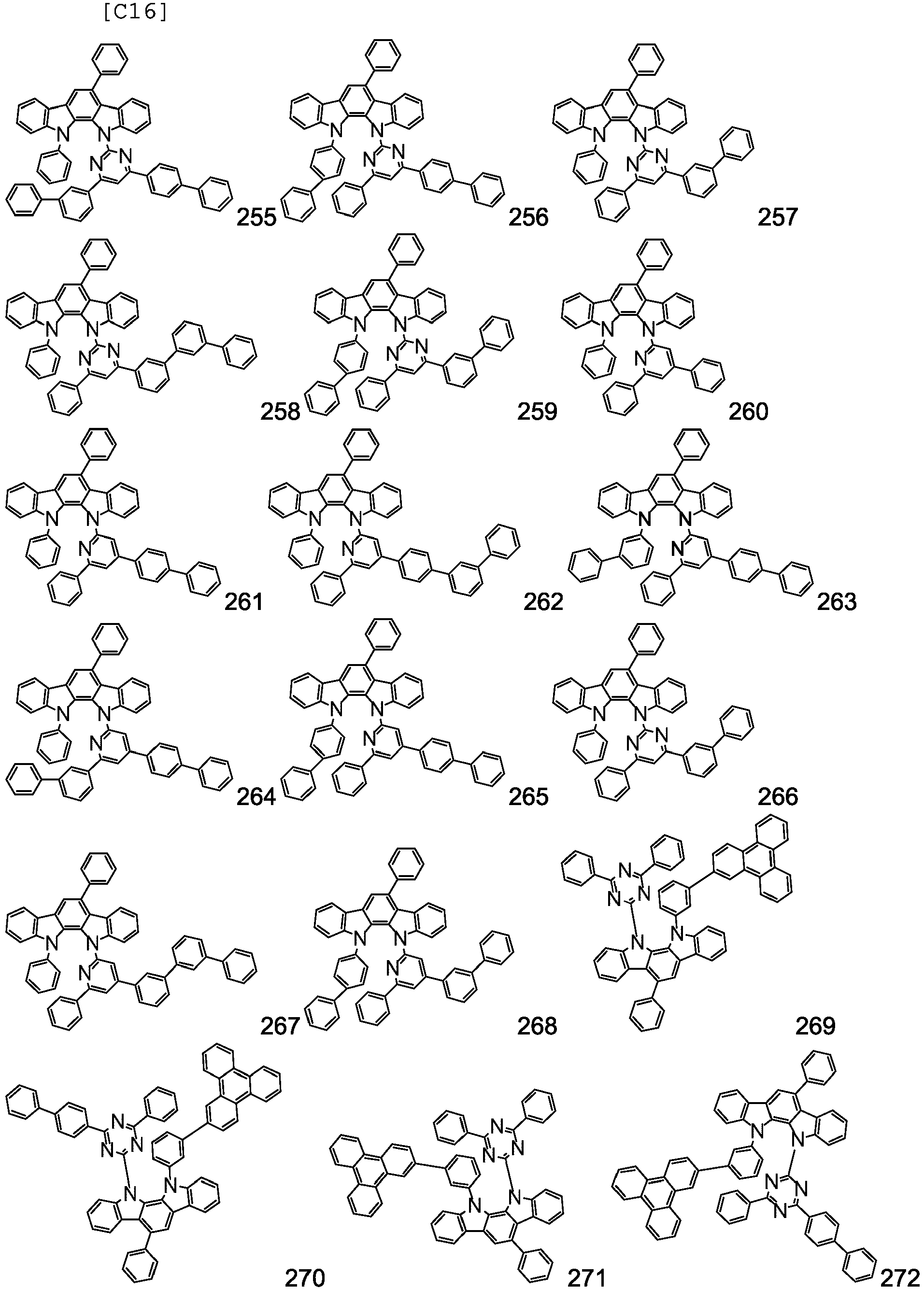

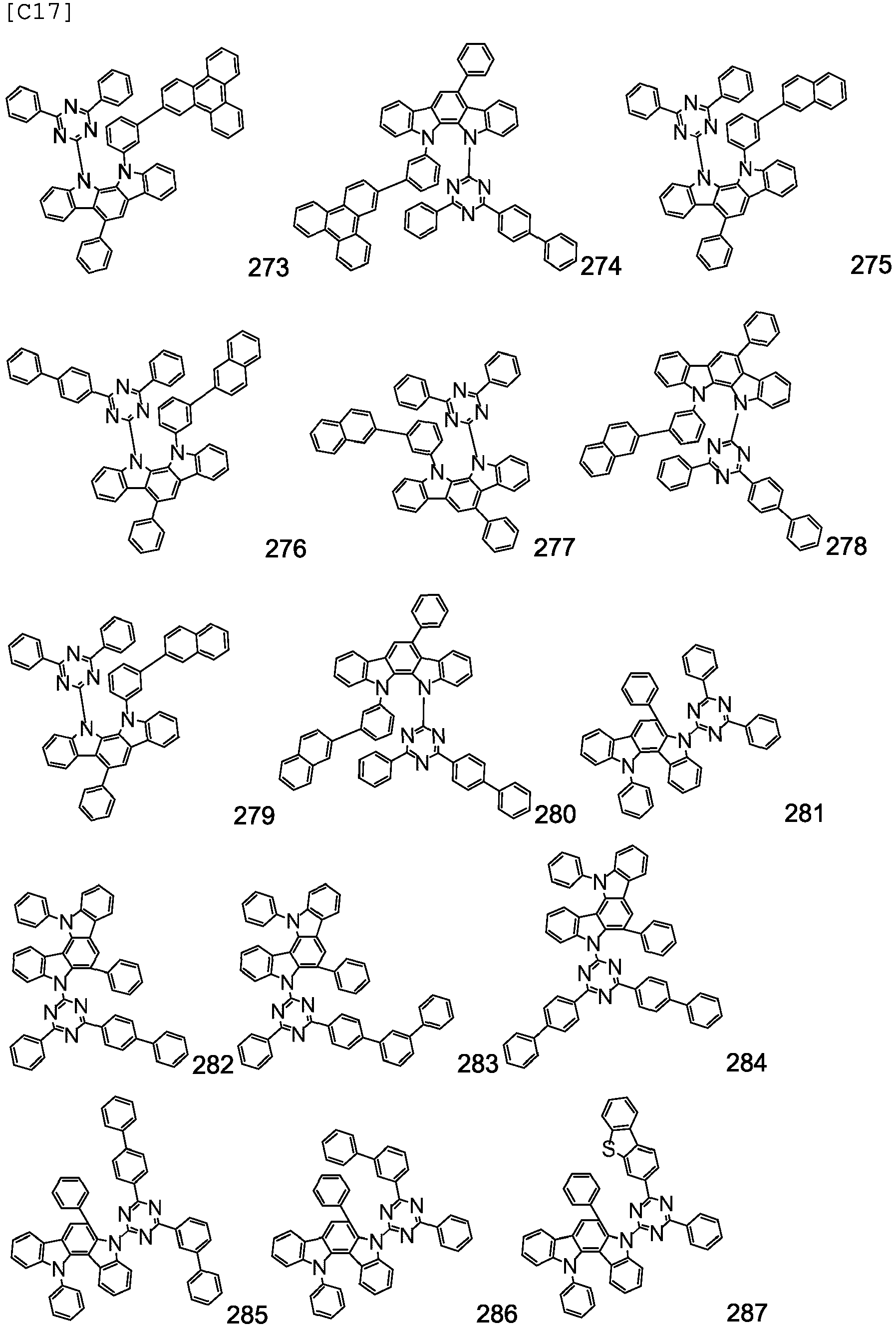

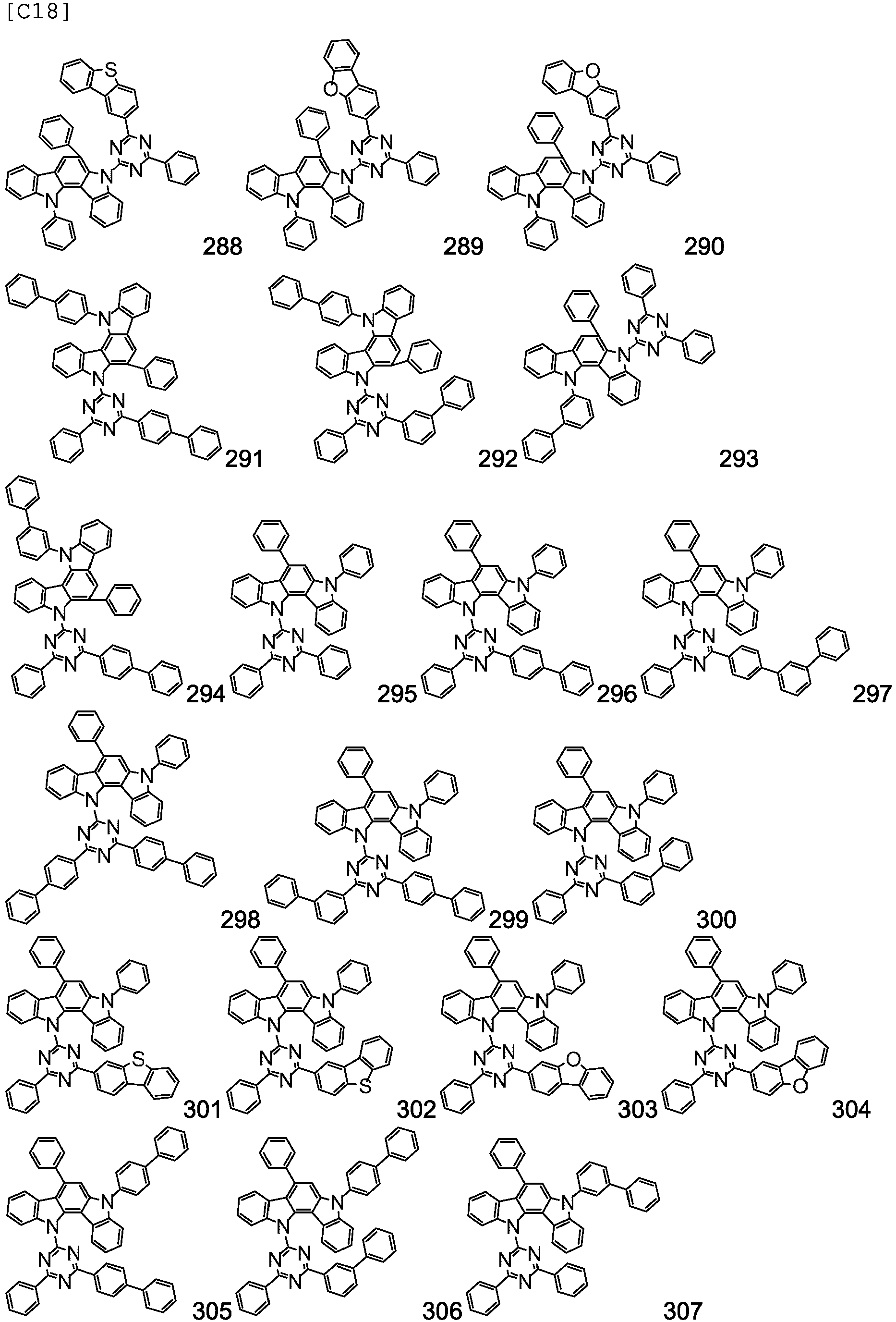

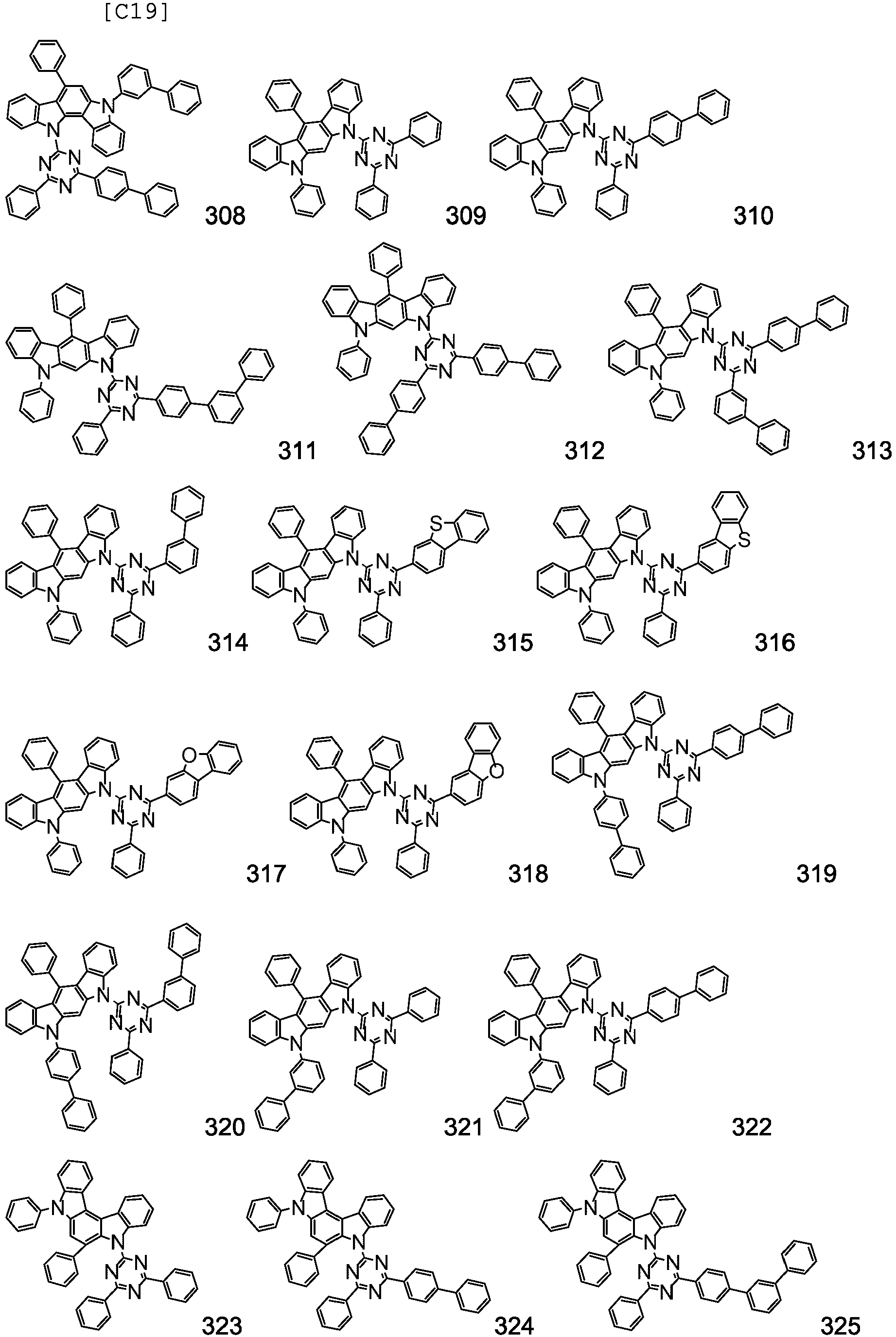

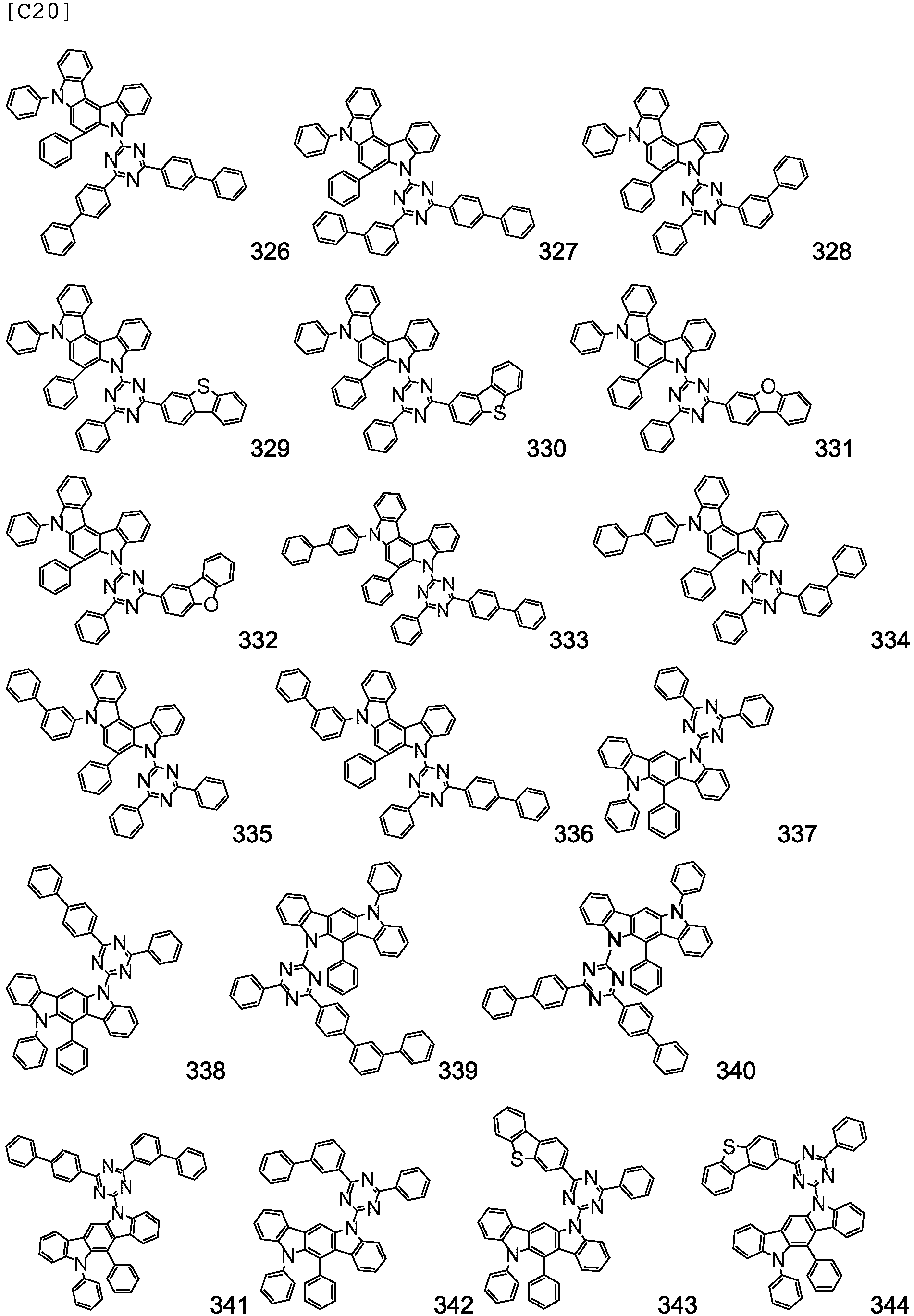

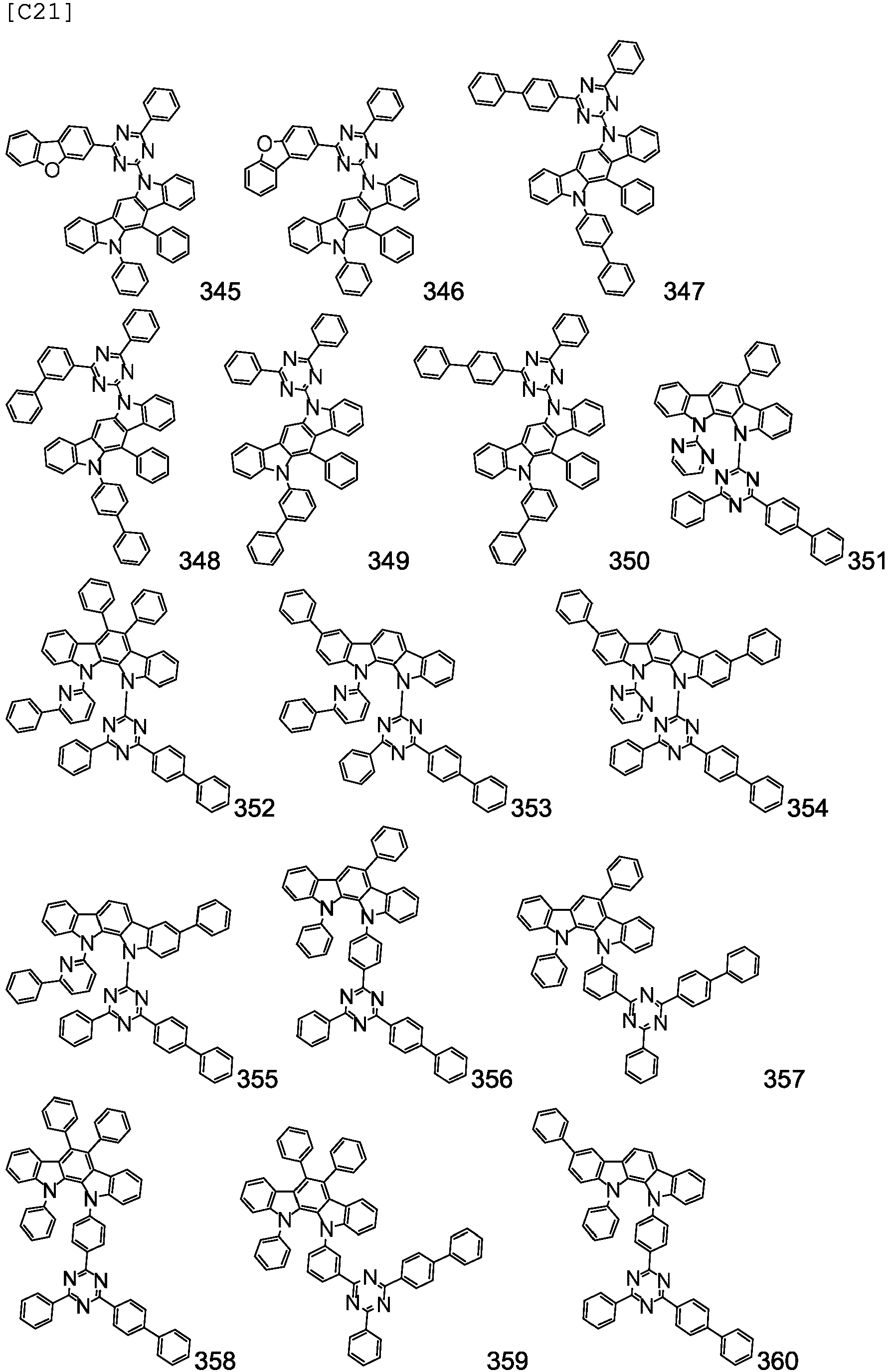

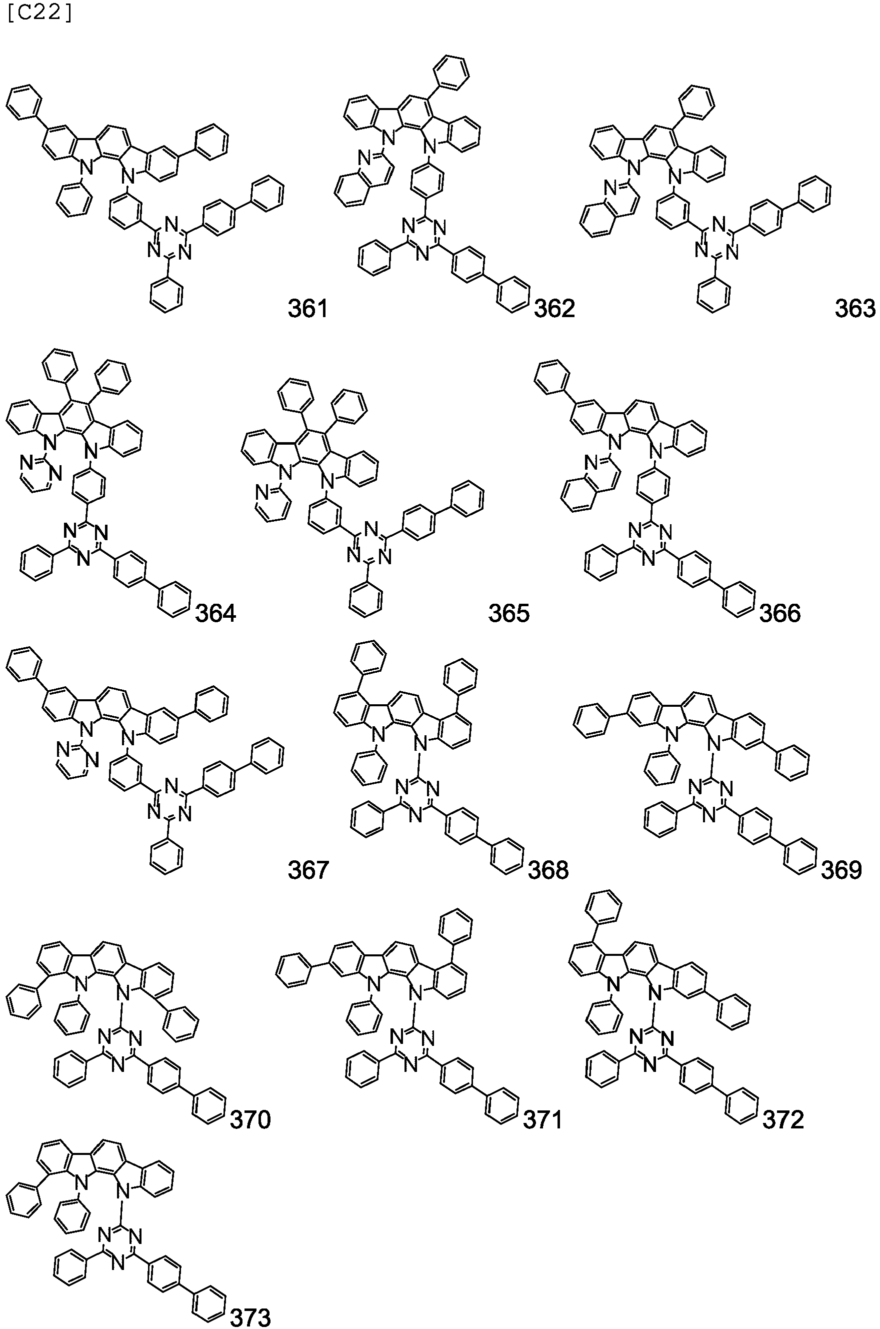

An organic EL device of the present invention has a plurality of organic layers between an anode and a cathode and includes the material for an organic EL device on at least one layer of the organic layers.

Another aspect of the material for an organic EL device of the present invention include the material for an organic EL device and also at least one of the compounds represented by the general formulas (8) to (10) in the same layer.

In the general formula (8), Ar⁴ and Ar⁵ each independently represent a substituted or unsubstituted aromatic hydrocarbon group having 6 to 30 carbon atoms, a substituted or unsubstituted aromatic heterocyclic group having 3 to 17 carbon atoms, or a substituted or unsubstituted linked aromatic group in which two to five of these aromatic rings are linked to each other, preferably an aromatic hydrocarbon group having 6 to 12 carbon atoms, or a substituted or unsubstituted linked aromatic group in which two to four of these aromatic hydrocarbon groups are linked to each other, and more preferably an aromatic hydrocarbon group having 6 to 10 carbon atoms or a substituted or unsubstituted linked aromatic group in which two to three of these aromatic hydrocarbon groups are linked to each other.

In the case of Ar⁴ and Ar⁵ being an unsubstituted aromatic hydrocarbon group, aromatic heterocyclic group, or linked aromatic group, specific examples thereof are the same as in the case of Ar². Preferred examples thereof include a group generated by removing one hydrogen from benzene, naphthalene, or compounds in which two to four of these are linked to each other. More preferred is a phenyl group, a biphenyl group, or a terphenyl group. The terphenyl group may be linked linearly or branched.

Specific examples of the compounds represented by the general formula (8) are shown below, but are not limited to these exemplified compounds.

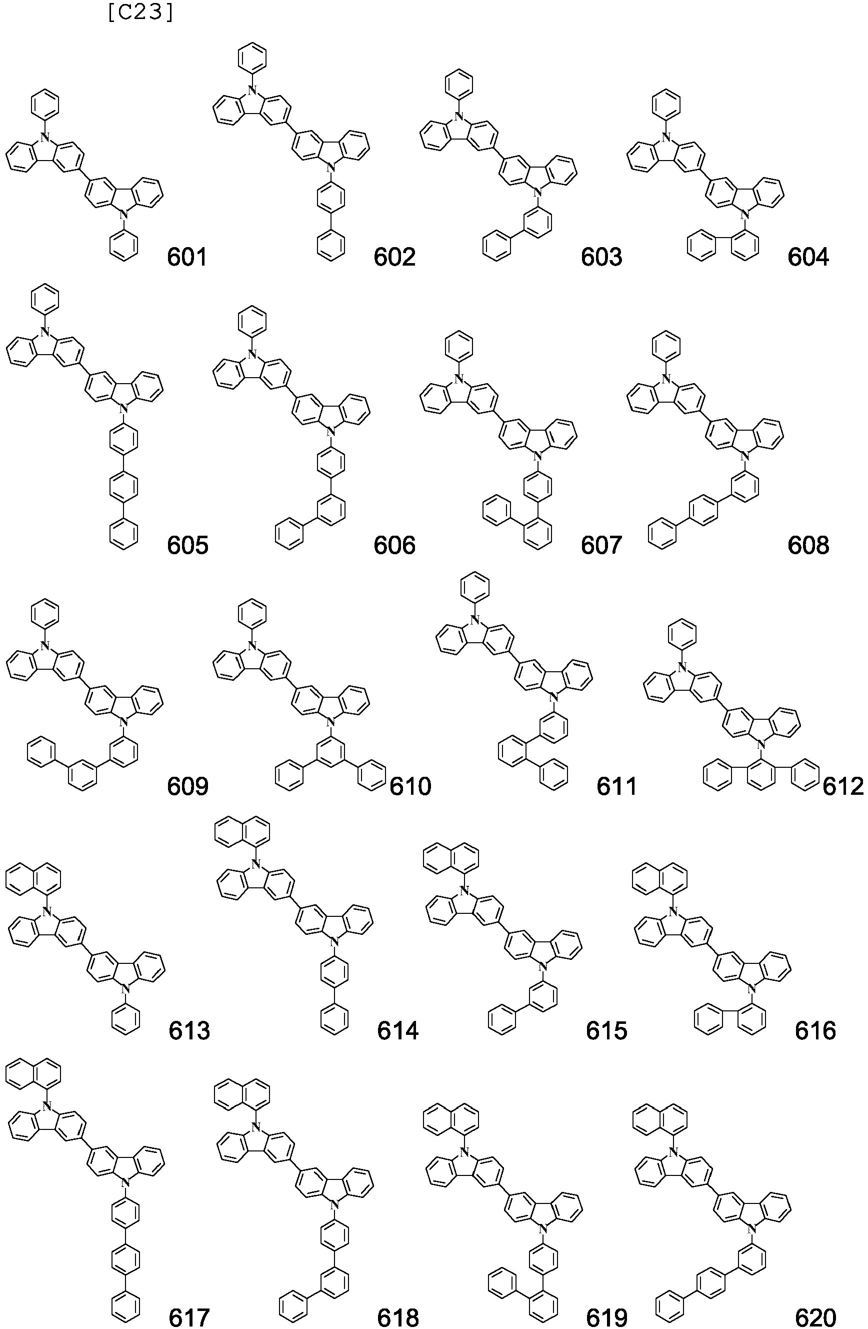

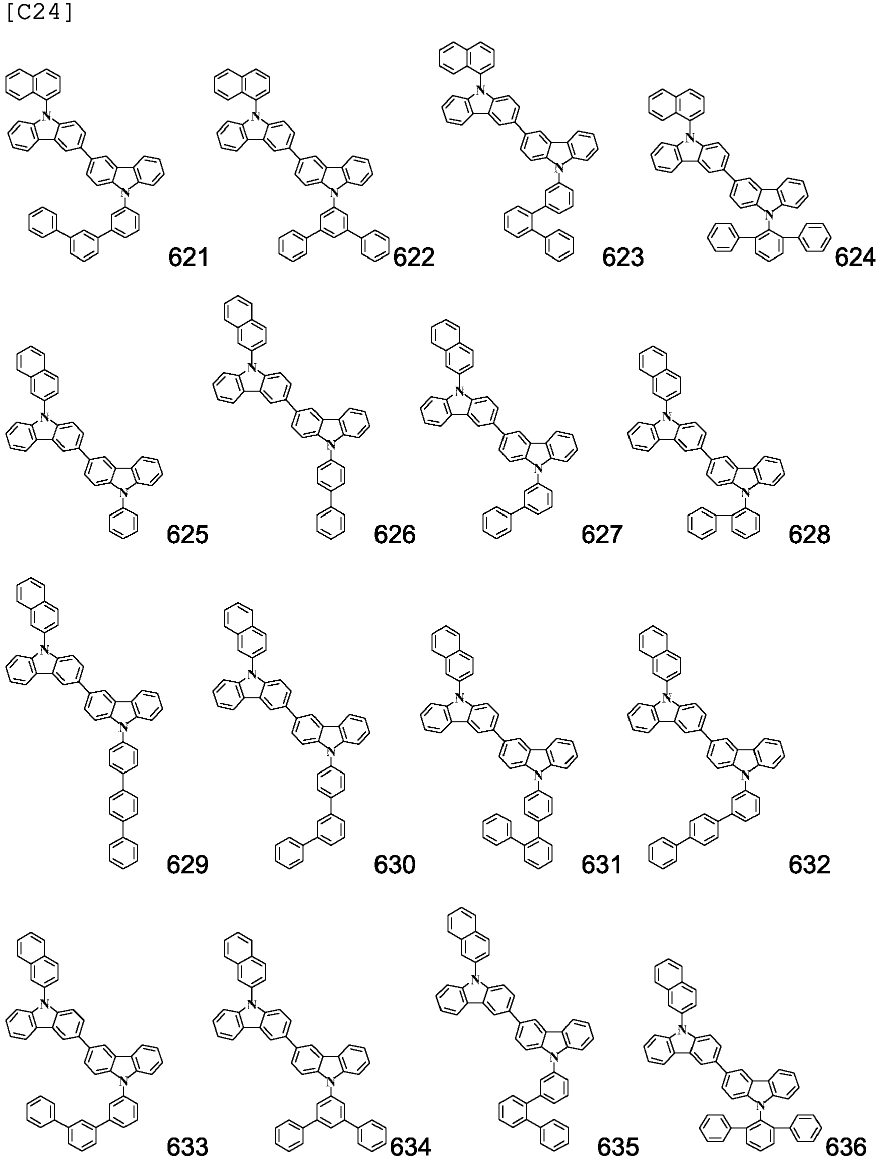

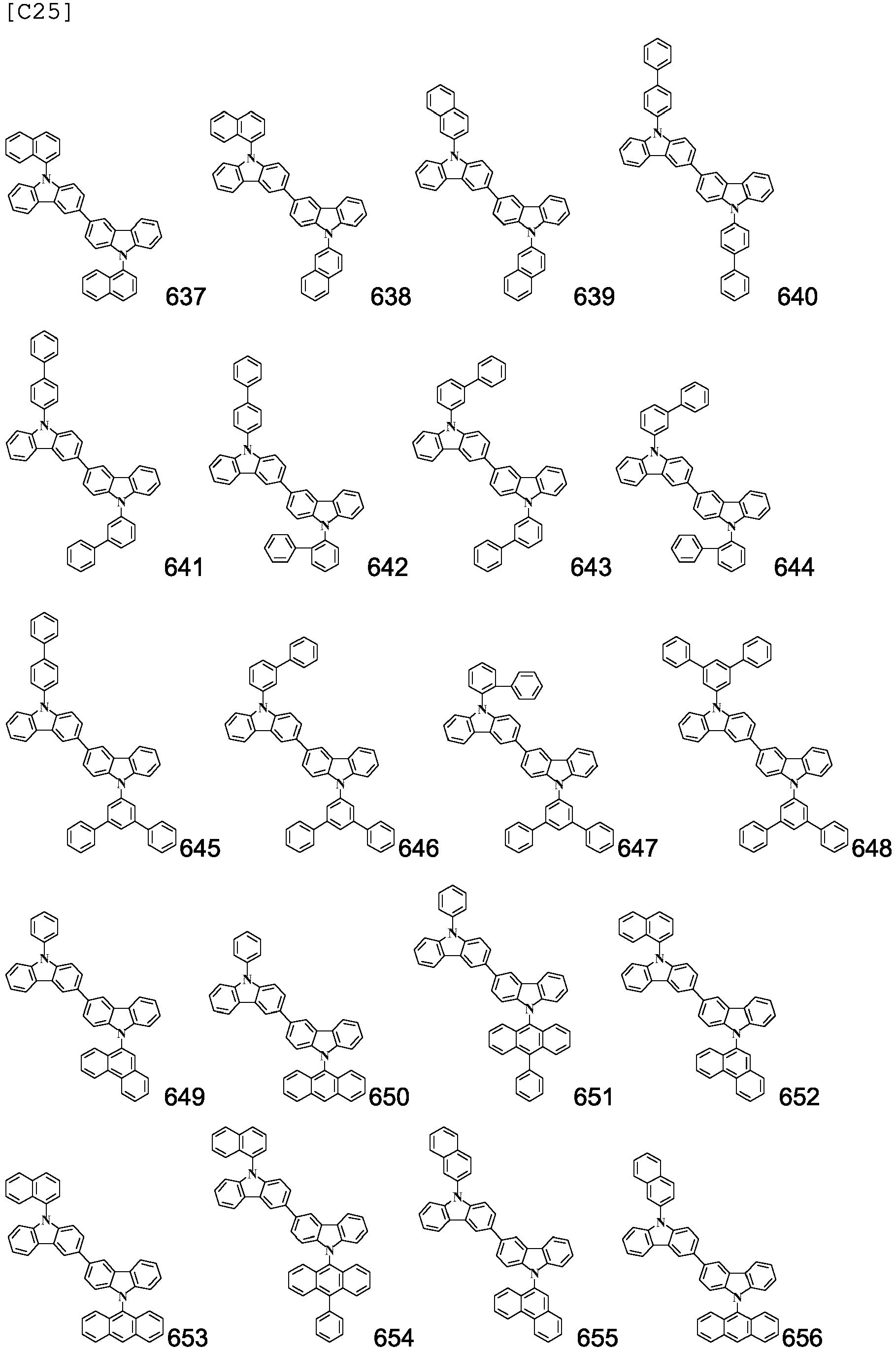

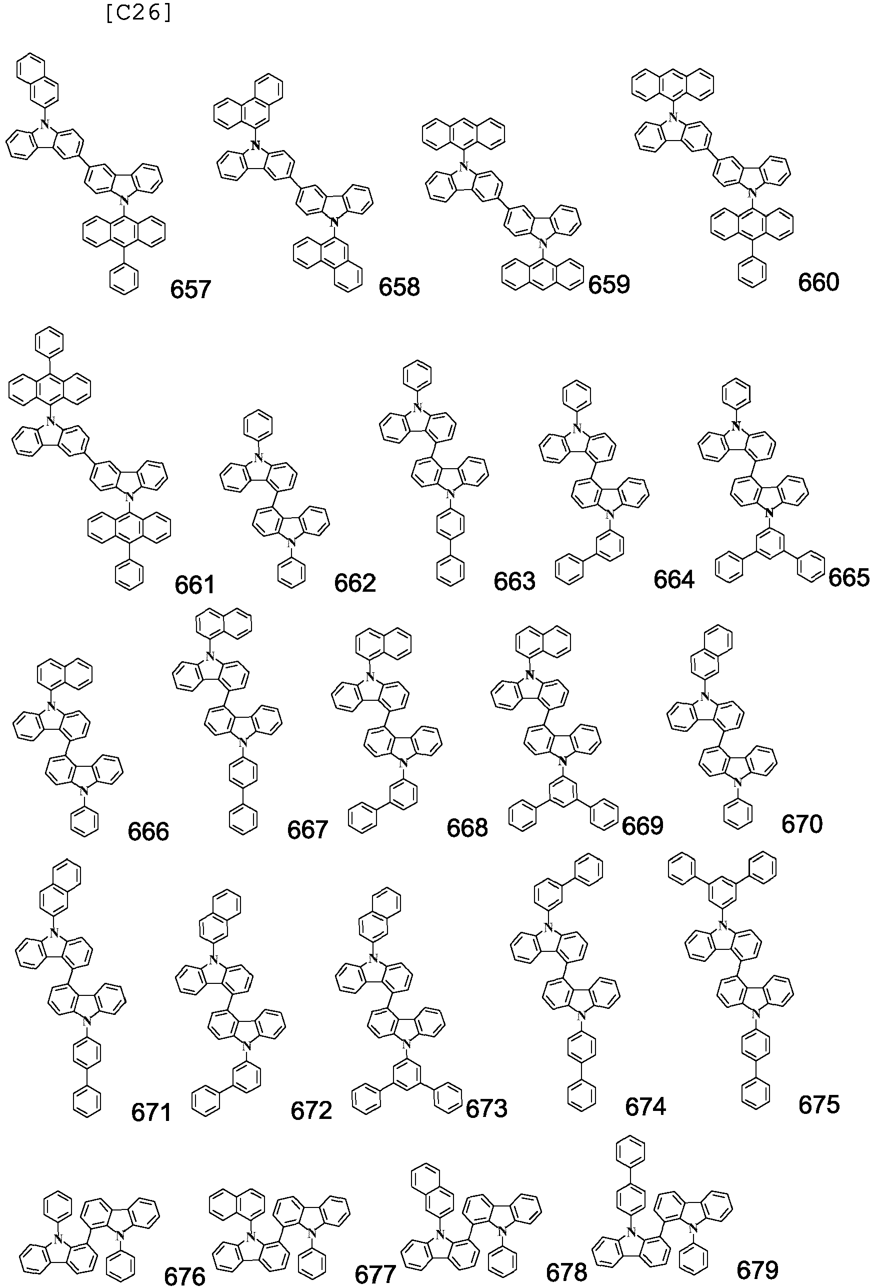

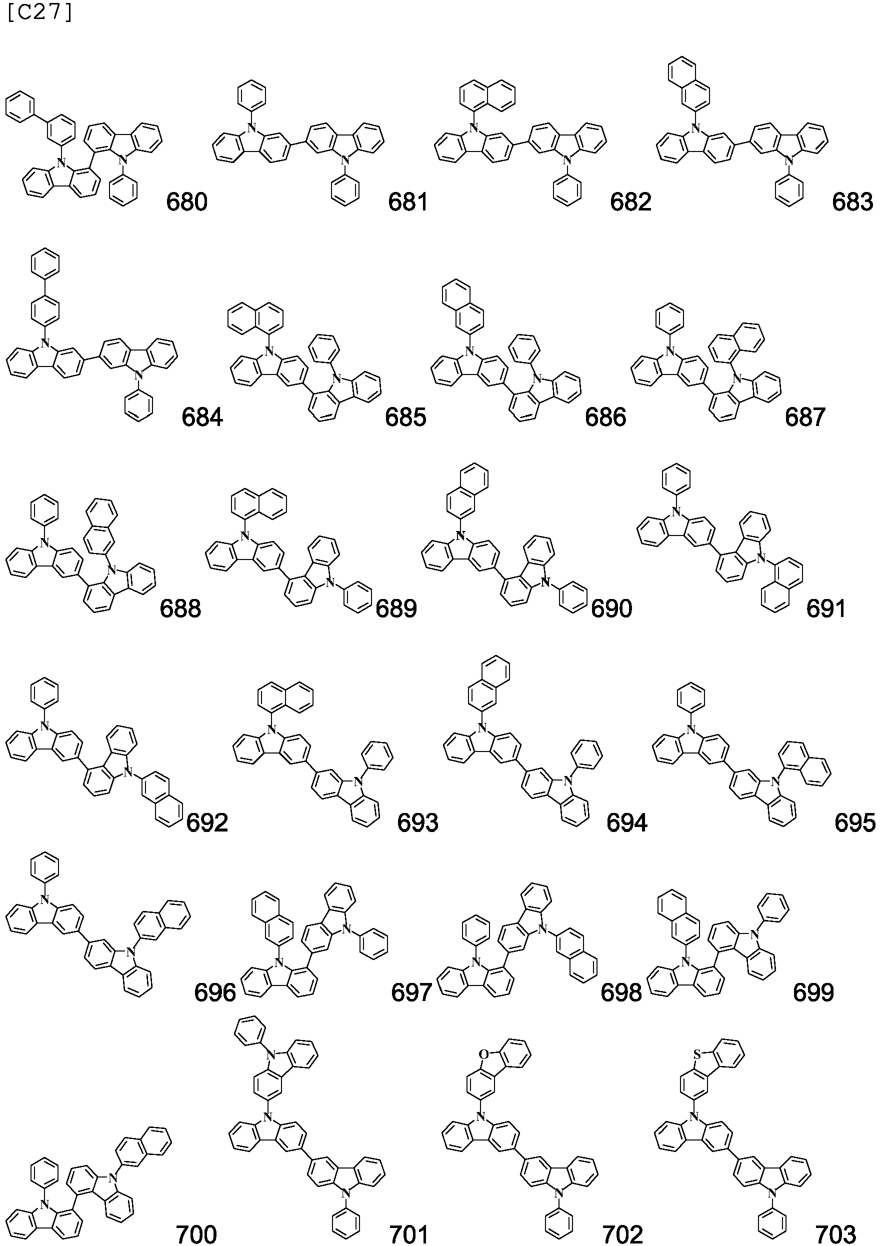

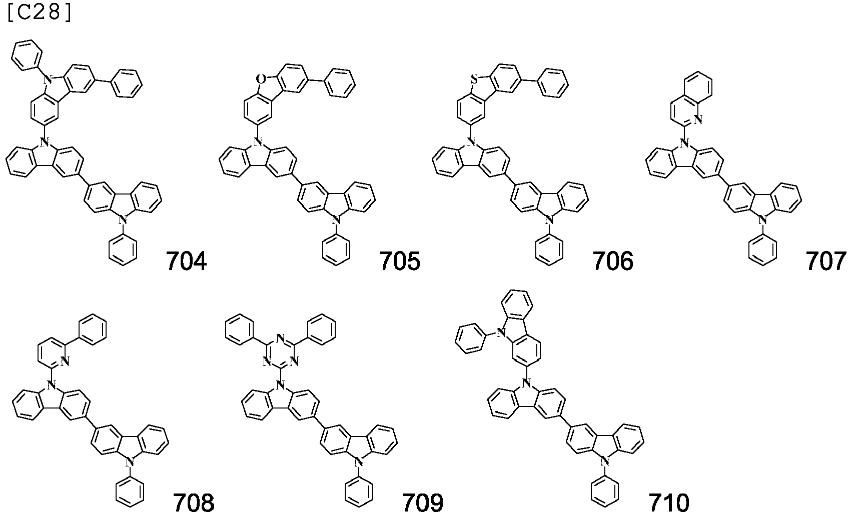

In the general formula (9), a ring B is a heterocycle represented by formula (9b) or (9c), and the ring B is fused to an adjacent ring at any position.

L³ independently represents a substituted or unsubstituted aromatic hydrocarbon group having 6 to 30 carbon atoms, or a substituted or unsubstituted aromatic heterocyclic group having 3 to 17 carbon atoms, preferably a substituted or unsubstituted aromatic hydrocarbon group having 6 to 24 carbon atoms, more preferably a substituted or unsubstituted aromatic hydrocarbon group having 6 to 18 carbon atoms, and further preferably a phenyl group. In the case of L³ being an unsubstituted aromatic hydrocarbon group or aromatic heterocyclic group, specific examples thereof are the same as in the case of Ar². Note that L³ is a divalent group.

X represents NAr⁸, O, or S, preferably NAr⁸ or O, and more preferably NAr⁸.

Ar⁶, Ar⁷, and Ar⁸ each independently represent a substituted or unsubstituted aromatic hydrocarbon group having 6 to 30 carbon atoms, a substituted or unsubstituted aromatic heterocyclic group having 3 to 17 carbon atoms, or a substituted or unsubstituted linked aromatic group in which two to five of these aromatic rings are linked to each other, preferably a substituted or unsubstituted aromatic hydrocarbon group having 6 to 24 carbon atoms, a substituted or unsubstituted aromatic heterocyclic group having 3 to 15 carbon atoms, or a substituted or unsubstituted linked aromatic group in which two to five of these aromatic rings are linked to each other, and more preferably a substituted or unsubstituted aromatic hydrocarbon group having 6 to 18 carbon atoms, or a substituted or unsubstituted linked aromatic group in which two to five of these aromatic hydrocarbon groups are linked to each other.

In the case of Ar⁶, Ar⁷, and Ar⁸ being an unsubstituted aromatic hydrocarbon group, aromatic heterocyclic group, or linked aromatic group, specific examples thereof are the same as in the case of Ar² and preferably include a group generated by removing one hydrogen from benzene, naphthalene, acenaphthene, acenaphthylene, azulene, anthracene, chrysene, pyrene, phenanthrene, triphenylene, fluorene, benzo[a]anthracene, tetracene, pentacene, hexacene, coronene, heptacene, pyridine, pyrimidine, triazine, thiophene, isothiazole, thiazole, pyridazine, pyrrole, pyrazole, imidazole, triazole, thiadiazole, pyrazine, furan, isoxazole, quinoline, isoquinoline, quinoxaline, quinazoline, thiadiazole, phthalazine, tetrazole, indole, benzofuran, benzothiophene, benzoxazole, benzothiazole, indazole, benzimidazole, benzotriazole, benzisothiazole, benzothiadiazole, purine, pyranone, coumarin, isocoumarin, chromone, dibenzofuran, dibenzothiophene, dibenzoselenophene, carbazole, or compounds in which two to five of these are linked to each other. More preferred examples thereof include a group generated by removing one hydrogen form benzene, naphthalene, acenaphthene, acenaphthylene, azulene, anthracene, chrysene, pyrene, phenanthrene, triphenylene, fluorene, benzo[a]anthracene, tetracene, or compounds in which two to five of these are linked to each other. Further preferred is a phenyl group, a biphenyl group, or a terphenyl group. The terphenyl group may be linked linearly or branched.

Ar⁶ preferably represents a substituted or unsubstituted aromatic hydrocarbon group having 6 to 30 carbon atoms, a substituted or unsubstituted aromatic heterocyclic group having 12 to 17 carbon atoms, or a substituted or unsubstituted linked aromatic group in which two to five of these aromatic rings are linked to each other, more preferably a substituted or unsubstituted aromatic hydrocarbon group having 6 to 24 carbon atoms, a substituted or unsubstituted aromatic heterocyclic group having 12 to 17 carbon atoms, or a substituted or unsubstituted linked aromatic group in which two to five of these aromatic rings are linked to each other, and further preferably a substituted or unsubstituted aromatic hydrocarbon group having 6 to 18 carbon atoms, or a substituted or unsubstituted linked aromatic group in which two to five of these aromatic hydrocarbon groups are linked to each other.

In the case of Ar⁶ being an unsubstituted aromatic heterocyclic group having 12 to 17 carbon atoms, specific examples thereof include a group generated from dibenzofuran, dibenzothiophene, dibenzoselenophene, or carbazole.

i and j each independently represent an integer of 0 to 3; preferably, i and j are 0 to 1; and more preferably, i is 0 and j is 1. However, i + j is an integer of 1 or more.

k and v represent the number of substitutions, k represents an integer of 0 to 3, and v independently represents an integer of 0 to 4; and preferably, k and v are 0 to 1; and more preferably, k and v are 0.

R¹ to R³ each independently represent a cyano group, an alkyl group having 1 to 20 carbon atoms, an aralkyl group having 7 to 38 carbon atoms, an alkenyl group having 2 to 20 carbon atoms, an alkynyl group having 2 to 20 carbon atoms, a dialkylamino group having 2 to 40 carbon atoms, a diarylamino group having 12 to 44 carbon atoms, a diaralkylamino group having 14 to 76 carbon atoms, an acyl group having 2 to 20 carbon atoms, an acyloxy group having 2 to 20 carbon atoms, an alkoxy group having 1 to 20 carbon atoms, an alkoxycarbonyl group having 2 to 20 carbon atoms, an alkoxycarbonyloxy group having 2 to 20 carbon atoms, an alkylsulfonyl group having 1 to 20 carbon atoms, a substituted or unsubstituted aromatic hydrocarbon group having 6 to 30 carbon atoms, or a substituted or unsubstituted aromatic heterocyclic group having 3 to 17 carbon atoms. Preferred is a substituted or unsubstituted aromatic hydrocarbon group having 6 to 24 carbon atoms, or a substituted or unsubstituted aromatic heterocyclic group having 3 to 17 carbon atoms, and more preferred is a substituted or unsubstituted aromatic hydrocarbon group having 6 to 18 carbon atoms, or an unsubstituted aromatic heterocyclic group having 3 to 12 carbon atoms.

In the case of R¹ to R³ being an alkyl group having 1 to 20 carbon atoms, an aralkyl group having 7 to 38 carbon atoms, an alkenyl group having 2 to 20 carbon atoms, an alkynyl group having 2 to 20 carbon atoms, a dialkylamino group having 2 to 40 carbon atoms, a diarylamino group having 12 to 44 carbon atoms, a diaralkylamino group having 14 to 76 carbon atoms, an acyl group having 2 to 20 carbon atoms, an acyloxy group having 2 to 20 carbon atoms, an alkoxy group having 1 to 20 carbon atoms, an alkoxycarbonyl group having 2 to 20 carbon atoms, an alkoxycarbonyloxy group having 2 to 20 carbon atoms, or an alkylsulfonyl group having 1 to 20 carbon atoms, specific examples thereof include alkyl groups such as methyl, ethyl, propyl, butyl, pentyl, cyclopentyl, hexyl, cyclohexyl, heptyl, octyl, nonyl, decyl, undecyl, dodecyl, tridecyl, tetradecyl, pentadecyl, hexadecyl, heptadecyl, octadecyl, nonadecyl, and icosyl, aralkyl groups such as phenylmethyl, phenylethyl, phenylicosyl, naphthylmethyl, anthranylmethyl, phenanthrenylmethyl, and pyrenylmethyl, alkenyl groups such as vinyl, propenyl, butenyl, pentenyl, decenyl, and icosenyl, alkynyl groups such as ethynyl, propargyl, butynyl, pentynyl, decynyl, and icosynyl, dialkylamino groups such as dimethylamino, ethylmethylamino, diethylamino, dipropylamino, dibutylamino, dipentynylamino, didecylamino, and diicosylamino, diarylamino groups such as diphenylamino, naphthylphenylamino, dinaphthylamino, dianthranylamino, diphenanthrenylamino, and dipyrenylamino, diaralkylamino groups such as diphenylmethylamino, diphenylethylamino, phenylmethylphenylethylamino, dinaphthylmethylamino, dianthranylmethylamino, and diphenanthrenylmethylamino, acyl groups such as acetyl, propionyl, butyryl, valeryl, and benzoyl, acyloxy groups such as acetyloxy, propionyloxy, butyryloxy, valeryloxy, and benzoyloxy, alkoxy groups such as methoxy, ethoxy, propoxy, butoxy, pentoxy, hexoxy, heptoxy, octoxy, nonyloxy, and decanyloxy, alkoxycarbonyl groups such as methoxycarbonyl, ethoxycarbonyl, propoxycarbonyl, butoxycarbonyl, and pentoxycarbonyl, alkoxycarbonyloxy groups such as methoxycarbonyloxy, ethoxycarbonyloxy, propoxycarbonyloxy, butoxycarbonyloxy, and pentoxycarbonyloxy, alkylsulfoxy groups such as methylsulfonyl, ethylsulfonyl, propylsulfonyl, butylsulfonyl, and pentylsulfonyl, and a cyano group, a nitro group, a fluoro group, and a tosyl group. Preferred is methyl, ethyl, propyl, butyl, pentyl, cyclopentyl, hexyl, cyclohexyl, heptyl, octyl, nonyl, decyl, undecyl, or dodecyl.

In the case of R¹ to R³ being an unsubstituted aromatic hydrocarbon group or aromatic heterocyclic group, specific examples thereof are the same as in the case of Ar². In the case of R¹ to R³ being an aromatic hydrocarbon group, they may also fall under the general formula (1); however, in this case, compounds in which j in the general formula (9) is 1 or more are treated as compounds of the general formula (9), and the other compounds are treated as compounds of the general formula (1), and not as the compounds that fall under both.

f and h each independently represent an integer of 0 to 4, and is preferably 0 to 1 and more preferably 0. g represents an integer of 0 to 2, and is preferably 0 to 1 and more preferably 0.

Specific examples of the compounds represented by the general formula (9) are shown below, but are not limited to these exemplified compounds.

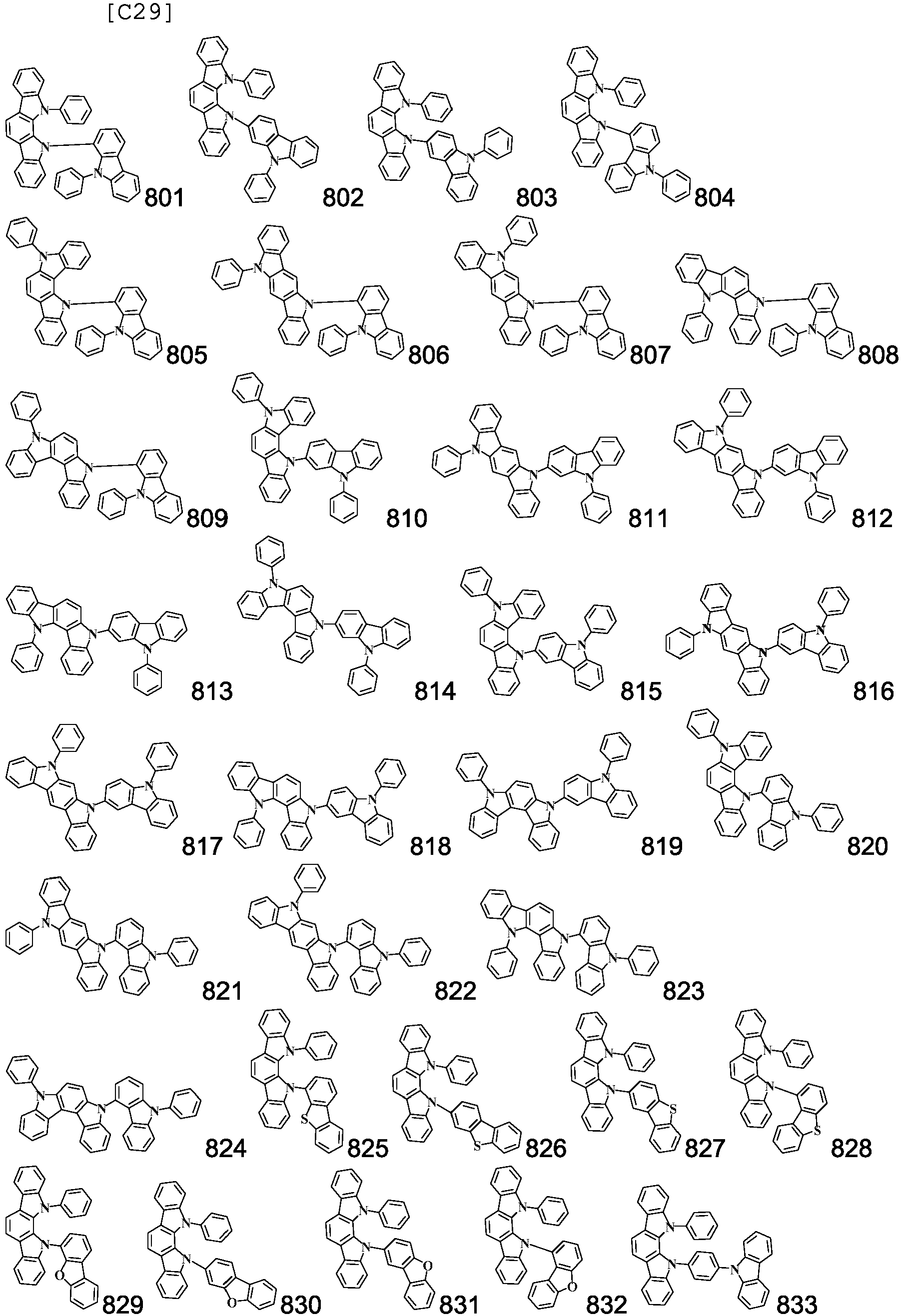

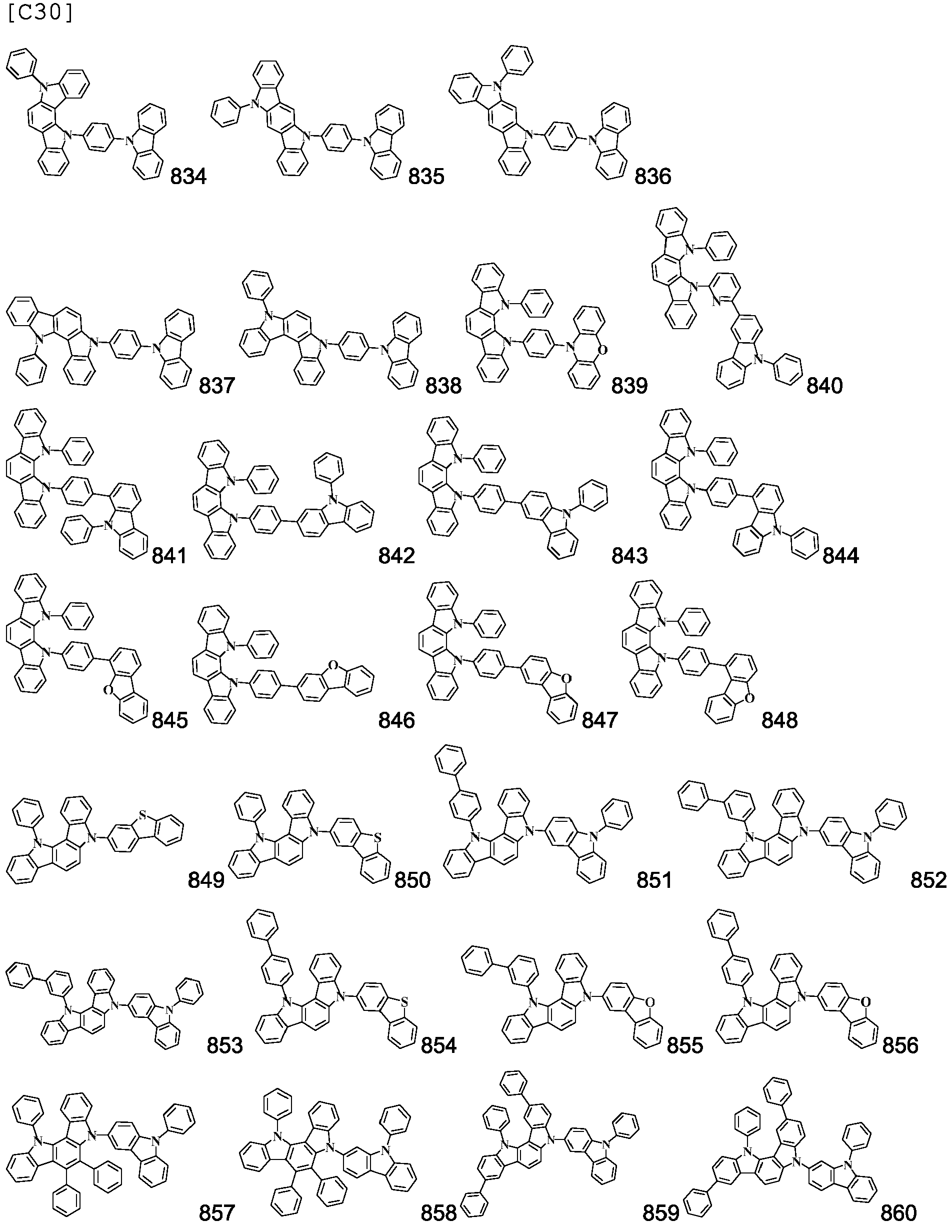

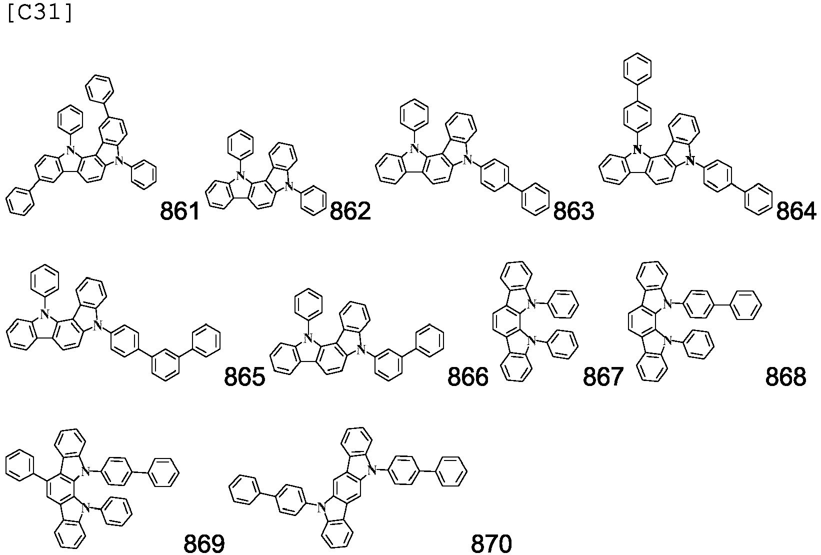

In the general formula (10), a ring D is a heterocycle represented by formula (10d), and the ring D is fused to an adjacent ring at any position.

Ar⁹ independently has the same meaning as Ar⁶ in the general formula (9).
R⁴ to R⁶ each independently have the same meaning as R¹ to R³ in the general formula (9).

l and n each independently represent an integer of 0 to 4, and is preferably 0 to 1 and more preferably 0.
m represents an integer of 0 to 2, and is preferably 0 to 1 and more preferably 0.

Ar¹⁰ represents a substituted or unsubstituted aromatic hydrocarbon group having 6 to 30 carbon atoms, a substituted or unsubstituted aromatic heterocyclic group having 3 to 17 carbon atoms, or a substituted or unsubstituted linked aromatic group in which two to five of them are linked to each other. Preferred is a substituted or unsubstituted aromatic hydrocarbon group having 6 to 24 carbon atoms, a substituted or unsubstituted aromatic heterocyclic group having 3 to 15 carbon atoms, or a substituted or unsubstituted linked aromatic group in which two to five of these aromatic rings are linked to each other, and more preferred is a substituted or unsubstituted aromatic hydrocarbon group having 6 to 18 carbon atoms, or a substituted or unsubstituted linked aromatic group in which two to five of these aromatic rings are linked to each other.

In the case of Ar¹⁰ being an unsubstituted aromatic hydrocarbon group or an unsubstituted aromatic heterocyclic group, specific examples thereof are the same as in the case of Ar⁷ and Ar⁸. Note that Ar¹⁰ is a divalent group.

Specific examples of the compounds represented by the general formula (10) are shown below, but are not limited to these exemplified compounds.

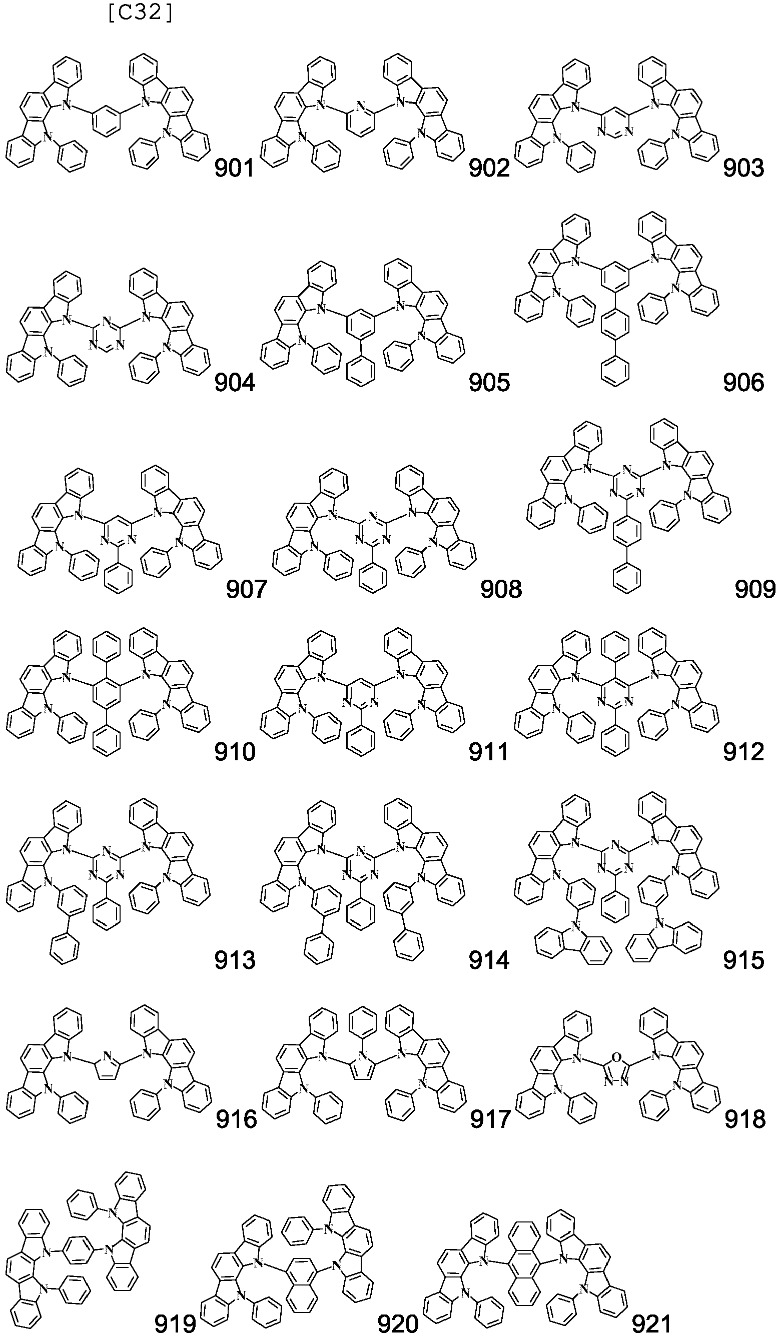

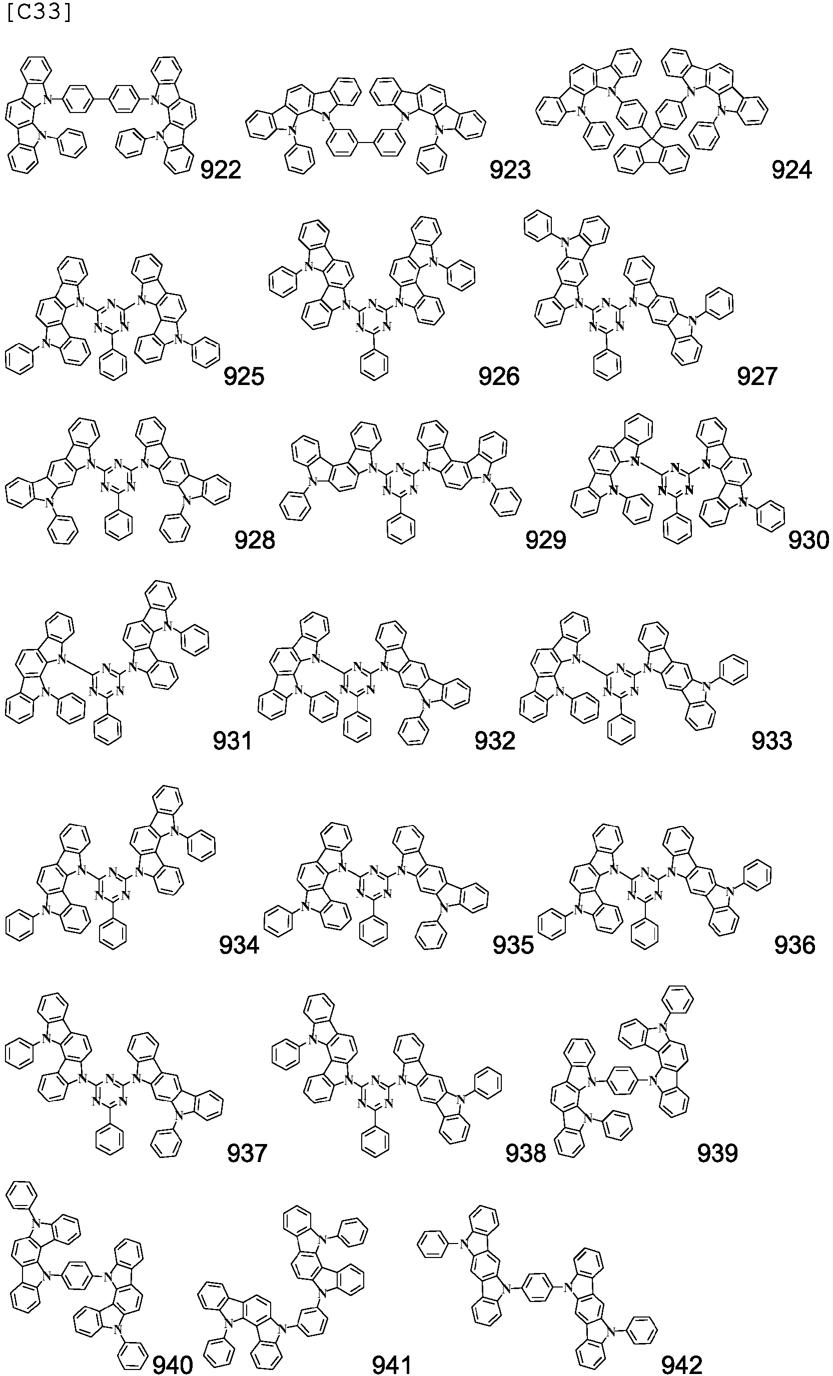

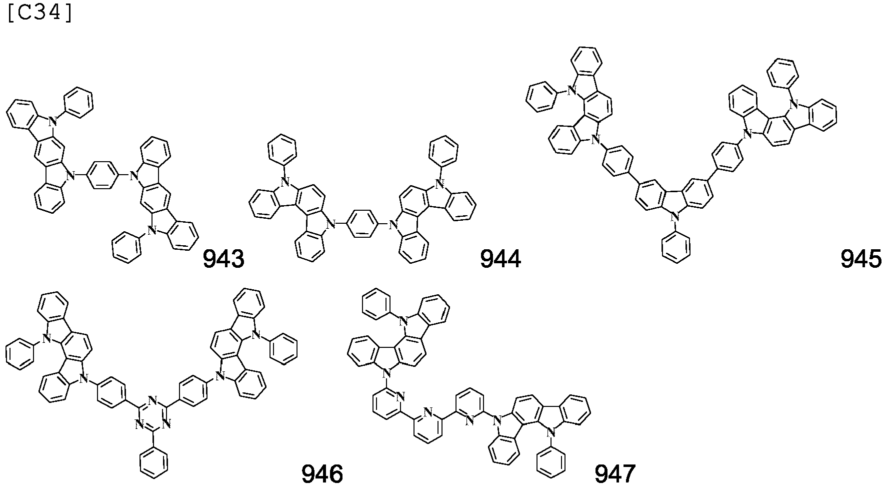

The material for an organic EL device of the present invention is contained in the organic layer, and this organic layer may be selected from the group comprising of a light-emitting layer, a hole injection layer, a hole transport layer, an electron transport layer, an electron injection layer, a hole blocking layer, and an electron blocking layer.

Preferred is a light-emitting layer, and the light-emitting layer may contain at least one light-emitting dopant.

If the material for an organic EL device of the present invention is contained in the light-emitting layer, it is desirable that the material be contained as a host. Advantageously, the material for an organic EL device of the present invention may be contained as the first host, and a compound selected from the compounds represented by the general formula (8), (9), or (10) as the second host.

The compounds, if used as the first host and the second host, can be used by, for example, vapor deposition from different individual vapor deposition sources, but the light-emitting layer is preferably formed by obtaining a premixture by premixing before vapor deposition to prepare a mixed composition for an organic EL device (also referred to as a premixture) and vapor-depositing the premixture simultaneously from one vapor deposition source. In this case, the premixture may be mixed with a light-emitting dopant material necessary for formation of a light-emitting layer, or another host to be used as necessary. However, when there is a large difference in temperatures to provide desired vapor pressure, vapor deposition may be performed from another vapor deposition source.

The mixed composition of the present invention contains the compound represented by the general formula (1) and the compound represented by any of the general formulas (8) to (10). Regarding these compounds, one kind thereof may be used, or two or more kinds thereof may be used. Preferably, the compound represented by the general formula (1) and the compound represented by the general formula (8) are contained. It is desirable that each compound in the mixed composition have a 50% weight reduction temperature within 20°C.

In addition, regarding the mixing ratio (weight ratio) between the first host and the second host, the proportion of the first host may be 10 to 70%, and is preferably more than 15% and less than 65%, and more preferably 20 to 60% based on the first host and the second host in total.

Next, the structure of the organic EL device of the present invention will be described by referring to the drawing, but the structure of the organic EL device of the present invention is not limited thereto.

Figure 1 is a cross-sectional view showing a structure example of an organic EL device generally used for the present invention, in which there are indicated a substrate 1, an anode 2, a hole injection layer 3, a hole transport layer 4, a light-emitting layer 5, an electron transport layer 6, and a cathode 7. The organic EL device of the present invention may have an exciton blocking layer adjacent to the light-emitting layer and may have an electron blocking layer between the light-emitting layer and the hole injection layer. The exciton blocking layer can be inserted into either of the cathode side and the cathode side of the light-emitting layer and inserted into both sides at the same time. The organic EL device of the present invention has the anode, the light-emitting layer, and the cathode as essential layers, and preferably has a hole injection transport layer and an electron injection transport layer in addition to the essential layers, and further preferably has a hole blocking layer between the light-emitting layer and the electron injection transport layer. Note that the hole injection transport layer refers to either or both of a hole injection layer and a hole transport layer, and the electron injection transport layer refers to either or both of an electron injection layer and an electron transport layer.

A structure reverse to that of Figure 1 is applicable, in which a cathode 7, an electron transport layer 6, a light-emitting layer 5, a hole transport layer 4, and an anode 2 are laminated on a substrate 1 in this order. In this case, layers may be added or omitted as necessary.

### - Substrate -

The organic EL device of the present invention is preferably supported on a substrate. The substrate is not particularly limited, and those conventionally used in organic EL devices may be used, and substrates made of, for example, glass, a transparent plastic, or quartz may be used.

### - Anode -

Regarding an anode material for an organic EL device, it is preferable to use a material of a metal, an alloy, an electrically conductive compound, and a mixture thereof, each having a large work function (4 eV or more). Specific examples of such an electrode material include a metal such as Au, and a conductive transparent material such as CuI, indium tin oxide (ITO), SnOz, and ZnO. In addition, an amorphous material such as IDIXO (In₂O₃-ZnO), which is capable of forming a transparent conductive film, may be used. Regarding the anode, such an electrode material is used to form a thin film by, for example, a vapor-deposition or sputtering method, and a desired shape pattern may be formed by a photolithographic method; or if the pattern accuracy is not particularly required (about 100 µm or more), a pattern may be formed via a desired shape mask when the electrode material is vapor-deposited or sputtered. Alternatively, when a coatable substance such as an organic conductive compound is used, a wet film formation method such as a printing method or a coating method may be used. For taking emitted light from the anode, it is desired to have a transmittance of more than 10%, and the sheet resistance for the anode is preferably several hundreds Ω/□ or less. The film thickness is selected usually within 10 to 1000 nm, preferably within 10 to 200 nm though depending on the material.

### - Cathode -

Meanwhile, regarding a cathode material, preferable to a material of a metal (an electron injection metal), an alloy, an electrically conductive compound, or a mixture thereof, each having a small work function (4 eV or less) are used. Specific examples of such an electrode material include sodium, a sodium-potassium alloy, magnesium, lithium, a magnesium/copper mixture, a magnesium/silver mixture, a magnesium/aluminum mixture, a magnesium/indium mixture, an aluminum/aluminum oxide (Al₂O₃) mixture, indium, a lithium/aluminum mixture, and a rare earth metal. Among these, from the viewpoint of the electron injectability and the durability against oxidation and the like, a mixture of an electron injection metal and a second metal which is a stable metal having a larger work function value is suitable, and examples thereof include a magnesium/silver mixture, a magnesium/aluminum mixture, a magnesium/indium mixture, an aluminum/aluminum oxide mixture, a lithium/aluminum mixture and aluminum. The cathode can be produced by forming a thin film by a method such as vapor-depositing or sputtering of such a cathode material. In addition, the sheet resistance of cathode is preferably several hundreds Ω/□ or less. The film thickness is selected usually within 10 nm to 5 µm, preferably within 50 to 200 nm. Note that for transmission of emitted light, if either one of the anode and cathode of the organic EL device is transparent or translucent, emission luminance is improved, which is convenient.

In addition, formation of a film of the above metal with a thickness of 1 to 20 nm on the cathode, followed by formation of a conductive transparent material described in the description on the anode thereon, enables production of a transparent or translucent cathode, and application of this enables production of a device wherein an anode and a cathode both have transmittance.

### - Light-emitting layer -

The light-emitting layer is a layer that emits light after excitons are generated when holes and electrons injected from the anode and the cathode, respectively, are recombined. As a light-emitting layer, an organic light-emitting dopant material and a host may be contained.

As a host, the first host and the second host may be used.

Regarding the compound represented by the general formula (1) as the first host, one kind thereof may be used, or two or more kinds thereof may be used. Similarly, regarding the carbazole compound or indolocarbazole compound represented by the general formulas (8) to (10) as the second host, one kind thereof may be used, or two or more kinds thereof may be used.

If necessary, one, or two or more other known host materials may be used in combination; however, it is preferable that an amount thereof to be used be 50 wt% or less, preferably 25 wt% or less based on the host materials in total.

The host and the premixture thereof may be in powder, stick, or granule form.

In the case of use of a plurality of kinds of hosts, such respective hosts can be vapor-deposited from different vapor deposition sources or can be simultaneously vapor-deposited from one vapor deposition source by premixing the hosts before vapor deposition to provide a premixture.

The premixing method is desirably a method that can allow for mixing as uniformly as possible, and examples thereof include pulverization and mixing, a heating and melting method under reduced pressure or under an atmosphere of an inert gas such as nitrogen, and sublimation, but not limited thereto.

When the first host and the second host are premixed and used, it is desirable that a difference in 50% weight reduction temperature (T₅₀) be small in order to produce an organic EL device having favorable characteristics with high reproducibility. The 50% weight reduction temperature is a temperature at which the weight is reduced by 50% when the temperature is raised to 550°C from room temperature at a rate of 10°C/min in TG-DTA measurement under a nitrogen stream reduced pressure (1 Pa). It is considered that vaporization due to evaporation or sublimation the most vigorously occurs around this temperature.

The difference in 50% weight reduction temperatures of the first host and the second host is preferably within 20°C, more preferably within 15°C. Regarding a premixing method, a known method such as pulverization and mixing can be used, and it is desirable to mix them as uniformly as possible.

When a phosphorescent dopant is used as a light-emitting dopant material, preferred is a phosphorescent dopant including an organic metal complex containing at least one metal selected from ruthenium, rhodium, palladium, silver, rhenium, osmium, iridium, platinum and gold. Specifically, iridium complexes described in J. Am. Chem. Soc. 2001, 123, 4304 and Japanese Translation of PCT International Application Publication No. 2013-53051 are preferably used, but the phosphorescent dopant is not limited thereto.

Regarding the phosphorescent dopant material, only one kind thereof may be contained in the light-emitting layer, or two or more kinds thereof may be contained. A content of the phosphorescent dopant material is preferably 0.1 to 30 wt% and more preferably 1 to 20 wt% with respect to the host material.

The phosphorescent dopant material is not particularly limited, and specific examples thereof include the following.

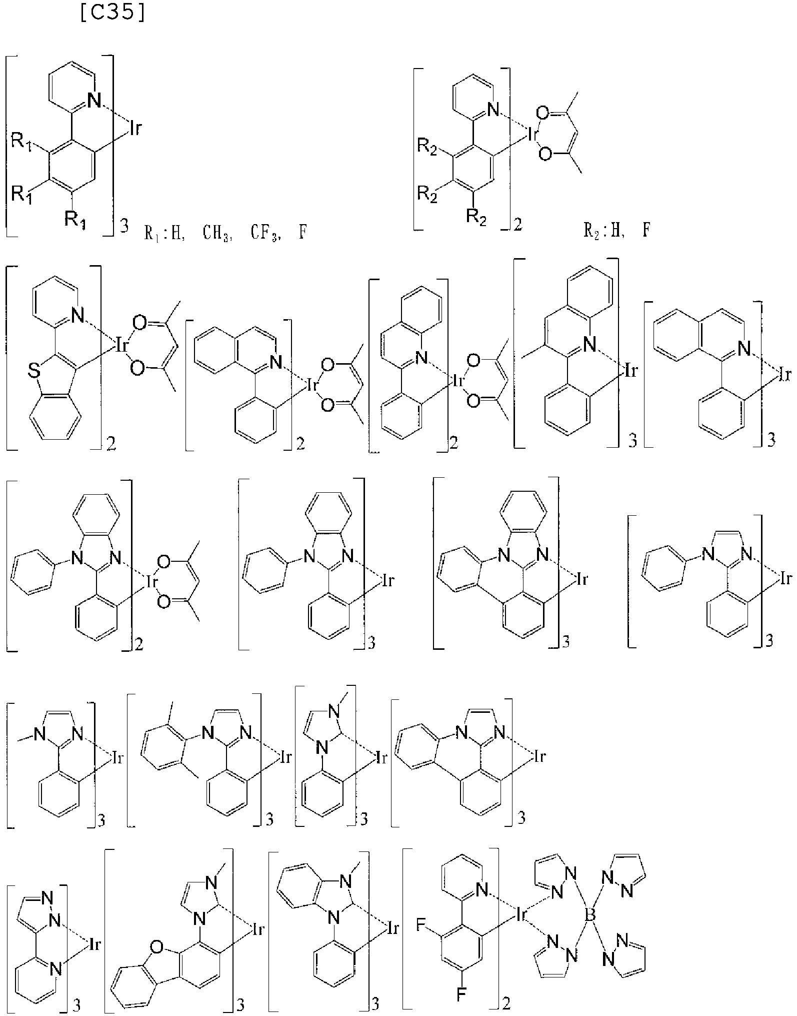

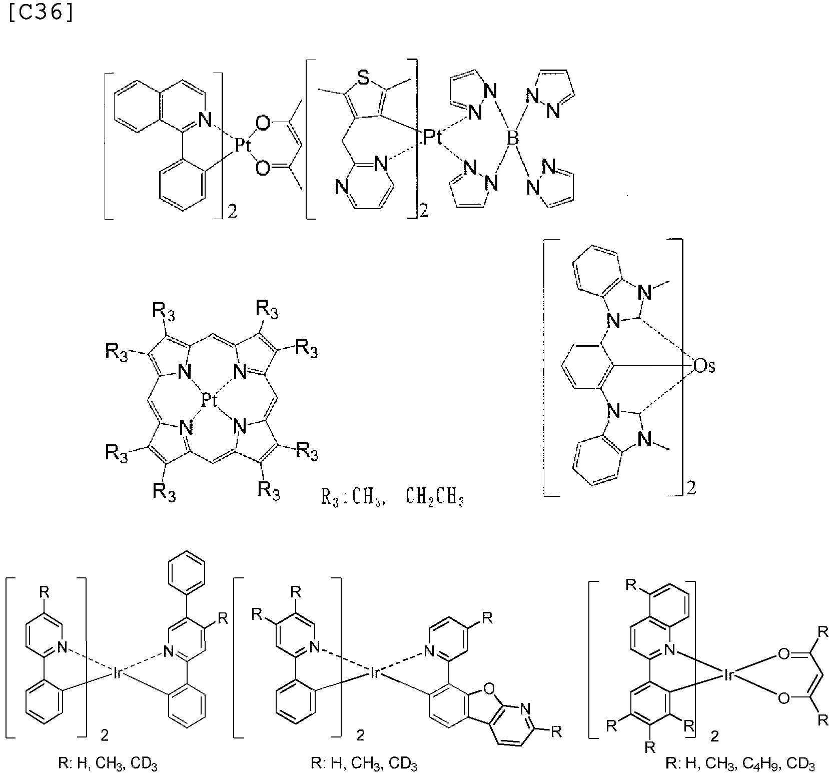

When a fluorescence-emitting dopant is used as the light-emitting dopant material, the fluorescence-emitting dopant is not particularly limited. Examples thereof include benzoxazole derivatives, benzothiazole derivatives, benzimidazole derivatives, styrylbenzene derivatives, polyphenyl derivatives, diphenylbutadiene derivatives, tetraphenyl butadiene derivatives, naphthalimido derivatives, coumarin derivatives, fused aromatic compounds, perinone derivatives, oxadiazole derivatives, oxazine derivatives, aldazine derivatives, pyrrolidine derivatives, cyclopentadiene derivatives, bisstyryl anthracene derivatives, quinacridone derivatives, pyrrolopyridine derivatives, thiadiazolopyridine derivatives, styrylamine derivatives, diketopyrrolopyrrole derivatives, aromatic dimethylidine compounds, metal complexes of 8-quinolinol derivatives or metal complexes of pyromethene derivatives, rare earth complexes, various metal complexes represented by transition metal complexes, polymer compounds such as polythiophene, polyphenylene, and polyphenylene vinylene, and organosilane derivatives. Preferred examples thereof include fused aromatic derivatives, styryl derivatives, diketopyrrolopyrrole derivatives, oxazine derivatives, pyromethene metal complexes, transition metal complexes, and lanthanoid complexes. More preferable examples thereof include naphthalene, pyrene, chrysene, triphenylene, benzo[c]phenanthrene, benzo[a]anthracene, pentacene, perylene, fluoranthene, acenaphthofluoranthene, dibenzo[a,j]anthracene, dibenzo[a,h]anthracene, benzo[a]naphthalene, hexacene, naphtho[2,1-f]isoquinoline, **α**-naphthaphenanthridine, phenanthrooxazole, quinolino[6,5-f]quinoline, and benzothiophanthrene. These may have an alkyl group, an aryl group, an aromatic heterocyclic group, or a diarylamino group as a substituent.

Regarding the fluorescence-emitting dopant material, only one kind thereof may be contained in the light-emitting layer, or two or more kinds thereof may be contained. A content of the fluorescence-emitting dopant material is preferably 0.1% to 20% and more preferably 1% to 10% with respect to the host material.

When a thermally activated delayed fluorescence-emitting dopant is used as the light-emitting dopant material, the thermally activated delayed fluorescence-emitting dopant is not particularly limited. Examples thereof include: metal complexes such as a tin complex and a copper complex; indolocarbazole derivatives described in WO2011/070963; cyanobenzene derivatives and carbazole derivatives described in Nature 2012, 492, 234; and phenazine derivatives, oxadiazole derivatives, triazole derivatives, sulfone derivatives, phenoxazine derivatives, and acridine derivatives described in Nature Photonics 2014, 8,326.

The thermally activated delayed fluorescence-emitting dopant material is not particularly limited, and specific examples thereof include the following.

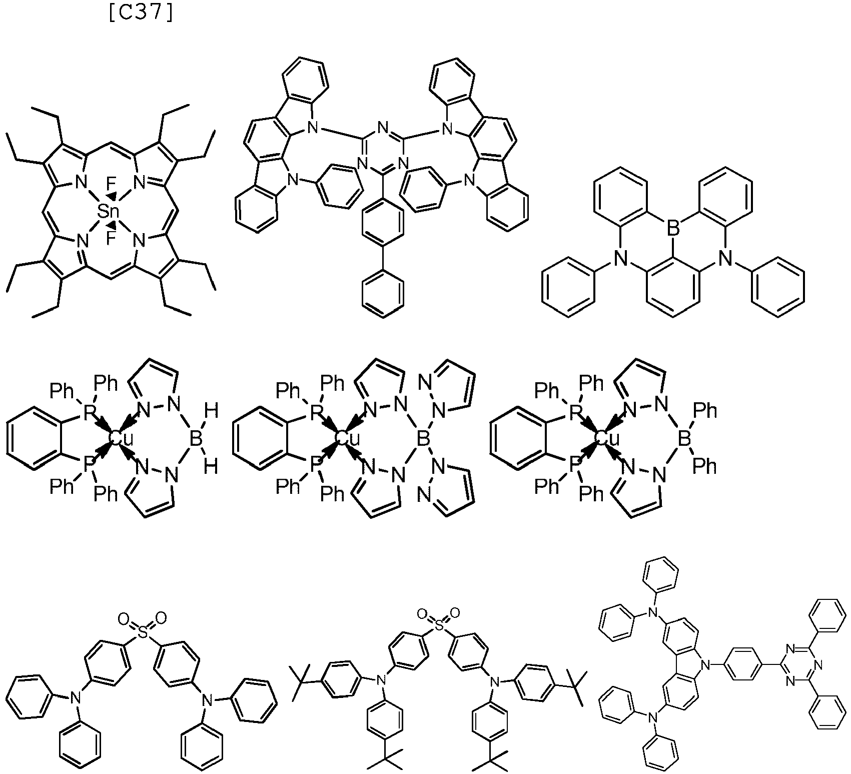

Regarding the thermally activated delayed fluorescence-emitting dopant material, only one kind thereof may be contained in the light-emitting layer, or two or more kinds thereof may be contained. In addition, the thermally activated delayed fluorescence-emitting dopant may be used by mixing with a phosphorescent dopant and a fluorescence-emitting dopant. A content of the thermally activated delayed fluorescence-emitting dopant material is preferably 0.1% to 50% and more preferably 1% to 30% with respect to the host material.

### - Injection Layer -

The injection layer is a layer that is provided between an electrode and an organic layer in order to lower a driving voltage and improve emission luminance, and includes a hole injection layer and an electron injection layer, and may be present between the anode and the light-emitting layer or the hole transport layer, and between the cathode and the light-emitting layer or the electron transport layer. The injection layer can be provided as necessary.

### - Hole Blocking Layer -

The hole blocking layer has a function of the electron transport layer in a broad sense, and is made of a hole blocking material having a function of transporting electrons and a significantly low ability to transport holes, and can block holes while transporting electrons, thereby improving a probability of recombining electrons and holes in the light-emitting layer.

For the hole blocking layer, a known hole blocking layer material can be used, but it is preferred for the layer to contain the compound represented by the general formula (1).

### - Electron Blocking Layer -

The electron blocking layer has a function of a hole transport layer in a broad sense and blocks electrons while transporting holes, thereby enabling a probability of recombining electrons and holes in the light-emitting layer to be improved.

Regarding the material of the electron blocking layer, a known electron blocking layer material can be used and a material of the hole transport layer to be described below can be used as necessary. A film thickness of the electron blocking layer is preferably 3 to 100 nm, and more preferably 5 to 30 nm.

### - Exciton Blocking Layer -

The exciton blocking layer is a layer for preventing excitons generated by recombination of holes and electrons in the light-emitting layer from being diffused in a charge transport layer, and insertion of this layer allows excitons to be efficiently confined in the light-emitting layer, enabling the luminous efficiency of the device to be improved. The exciton blocking layer can be inserted, in a device having two or more light-emitting layers adjacent to each other, between two adjacent light-emitting layers.

Regarding the material of the exciton blocking layer, a known exciton blocking layer material can be used. Examples thereof include 1,3-dicarbazolyl benzene (mCP) and bis(2-methyl-8-quinolinolato)-4-phenylphenolato aluminum (III) (BAlq) .

### - Hole Transport Layer -

The hole transport layer is made of a hole transport material having a function of transporting holes, and the hole transport layer can be provided as a single layer or a plurality of layers.

The hole transport material has either hole injection, transport properties or electron barrier properties, and may be an organic material or an inorganic material. For the hole transport layer, any one selected from conventionally known compounds can be used. Examples of such a hole transport material include porphyrin derivatives, arylamine derivatives, triazole derivatives, oxadiazole derivatives, imidazole derivatives, polyarylalkane derivatives, pyrazoline derivatives and pyrazolone derivatives, phenylenediamine derivatives, arylamine derivatives, amino-substituted chalcone derivatives, oxazole derivatives, styryl anthracene derivatives, fluorenone derivatives, hydrazone derivatives, stilbene derivatives, silazane derivatives, an aniline copolymer, and a conductive polymer oligomer, and particularly a thiophene oligomer. Use of porphyrin derivatives, arylamine derivatives, or styrylamine derivatives preferred. Use of arylamine compounds is more preferred.

### - Electron Transport Layer -

The electron transport layer is made of a material having a function of transporting electrons, and the electron transport layer can be provided as a single layer or a plurality of layers.

The electron transport material (which may also serve as a hole blocking material) may have a function of transferring electrons injected from the cathode to the light-emitting layer. For the electron transport layer, any one selected from conventionally known compounds can be used, and examples thereof include polycyclic aromatic derivatives such as naphthalene, anthracene, and phenanthroline, tris(8-quinolinolato)aluminum(III) derivatives, phosphine oxide derivatives, nitro-substituted fluorene derivatives, diphenylquinone derivatives, thiopyrandioxide derivatives, carbodiimide, fluorenylidene methane derivatives, anthraquinodimethane and anthrone derivatives, bipyridine derivatives, quinoline derivatives, oxadiazole derivatives, benzimidazole derivatives, benzothiazole derivatives, and indolocarbazole derivatives. In addition, a polymer material in which the above material is introduced into a polymer chain or the above material is used for a main chain of a polymer can be used.

### Examples

Hereafter, the present invention will be described in detail by referring to Examples, but the present invention is not limited these Examples and can be implemented in various forms without departing from the gist thereof.

### Synthesis Example 1

Compound 129 was synthesized in accordance with the next reaction formula.

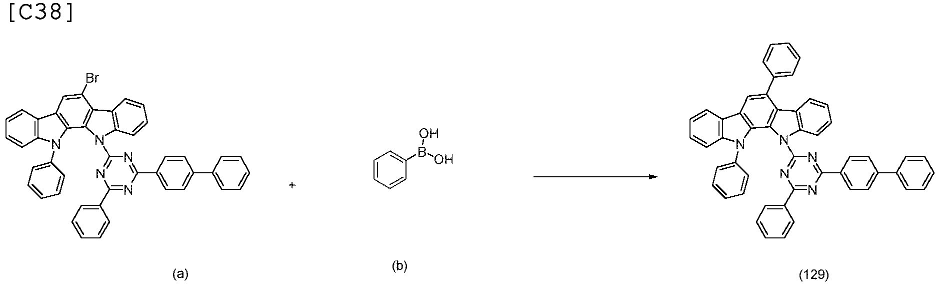

Under a nitrogen atmosphere, 10.0 g (13.9 mmol) of intermediate (a), 2.2 g (18.1 mmol) of compound (b), 0.5 g (0.4 mmol) of tetrakis(triphenylphosphine)palladium(0), 5.8 g of potassium carbonate, 300 ml of m-xylene, 30 ml of ethanol, and 30 ml of distilled water were added and stirred for 3 hours while being heating at 100°C. After the mixture was cooled to room temperature, distilled water (50 ml) was added thereto, and the organic layer was separated and concentrated to dryness. The resulting solid was purified by silica gel column chromatography and crystallization to give 5.3 g (7.4 mmol, 53.2% yield) of compound 129 as a yellow solid compound (APCI-TOFMS, m/z 716 [M+H]⁺).

### Synthesis Example 2

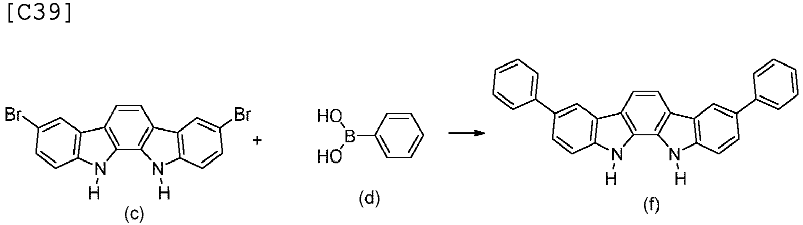

Under a nitrogen atmosphere, 10.0 g (24.2 mmol) of intermediate (c), 6.2 g (50.7 mmol) of compound (d), 0.8 g (0.7 mmol) of tetrakis(triphenylphosphine)palladium(0), 10.0 g of potassium carbonate, 300 ml of m-xylene, 30 ml of ethanol, and 30 ml of distilled water were added and stirred for 3 hours while being heating at 100°C. After the mixture was cooled to room temperature, distilled water (200 ml) was added thereto, and the precipitated solid was collected by filtration. The resulting solid was purified by crystallization to give 5.5 g (13.4 mmol, 55.8% yield) of intermediate (f) (APCI-TOFMS, m/z 409 [M+H]⁺).

### Synthesis Example 3

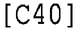

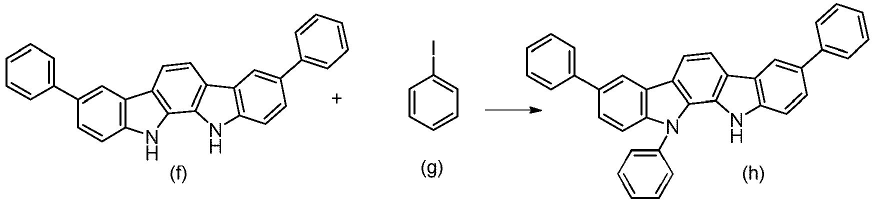

Under a nitrogen atmosphere, 10.0 g (24.5 mmol) of intermediate (f), 7.5 g (36.7 mmol) of compound (g), 0.9 g (4.9 mmol) of CuI, 26.0 g (120.0 mmol) of tripotassium phosphate, 300 ml of 1,4-dioxane, 1.1 g (9.8 mmol) of trans-1,2-cyclohexanediamine were added and stirred for 6 hours under heating at reflux. After the mixture was cooled to room temperature, the liquid layer obtained by solid-liquid separation was concentrated to dryness.

The resulting solid was purified by silica gel column chromatography to give 5.2 g (10.7 mmol, 43.8% yield) of intermediate (h) (APCI-TOFMS, m/z 485 [M+H]⁺).

### Synthesis Example 4

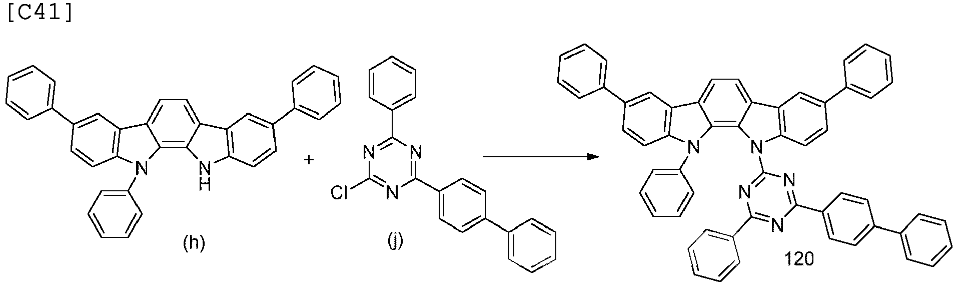

Under a nitrogen atmosphere, 1.0 g (24.8 mmol) of 60 wt% sodium hydride was added to 200 g of N,N'-dimethylacetamide to prepare a suspension. To this was added 10.0 g (20.6 mmol) of intermediate (h), the mixture was stirred for 1 hour and then cooled to 5°C. To this was added 9.2 g (26.8 mmol) of compound (i), then the mixture was restored to room temperature and stirred for 4 hours. The reaction solution was added to a solution of ethanol (100 ml) mixed with distilled water (200 ml) while being stirred, and the resulting precipitated solid was collected by filtration. The solid was purified by silica gel column chromatography and crystallization to give 7.1 g (9.0 mmol, 43.4% yield) of compound 120 as a yellow solid compound (APCI-TOFMS, m/z 792 [M+H]⁺).

Compounds 128, 155, 112, 122, and 156 were synthesized according to the synthesis examples. Compounds A, B, C, D and E were also synthesized for comparison.

### Example 1

On a glass substrate on which an anode made of ITO with a film thickness of 110 nm was formed, respective thin films were laminated by a vacuum evaporation method at a degree of vacuum of 4.0 × 10⁻⁵ Pa. First, CuPc was formed with a thickness of 25 nm as a hole injection layer on ITO, and next, NPD was formed with a thickness of 30 nm as a hole transport layer. Next, HT-1 was formed with a thickness of 10 nm as an electron blocking layer. Then, compound 128 as a host material and Ir(ppy)₃ as a light-emitting dopant were co-vapor-deposited from different vapor deposition sources, respectively, to form a light-emitting layer with a thickness of 40 nm. In this case, the concentration of Ir(ppy)₃ was 10 wt%. Furthermore, H-3 was formed with a thickness of 10 nm as a hole blocking layer. Next, ET-1 was formed with a thickness of 10 nm as an electron transport layer. Further, LiF was formed with a thickness of 1 nm as an electron injection layer on the electron transport layer. Finally, Al was formed with a thickness of 70 nm as a cathode on the electron injection layer to produce an organic EL device.

When an external power supply was connected to the obtained organic EL devices and a DC voltage was applied, an emission spectrum with a maximum wavelength of 517 nm was observed, and it was thus found that light emission from Ir(ppy)₃ was obtained.

### Examples 2 to 7

Organic EL devices were produced in the same manner as in Example 1 except that compounds 155, 112, 120, 122, 129, and 156 were used as the host material of the light-emitting layer instead of compound 128 of Example 1. When a DC voltage was applied to the obtained organic EL devices, an emission spectrum with a maximum wavelength of 517 nm was observed.

### Comparative Examples 1 to 4

Organic EL devices were produced in the same manner as in Example 1 except that compound A, B, C, or D was used as the host material of the light-emitting layer instead of that of Example 1. When an external power supply was connected to the obtained organic EL devices and a DC voltage was applied, an emission spectrum with a maximum wavelength of 517 nm was observed.

Evaluation results of the produced organic EL devices are shown in Table 1. In the tables, the luminance, driving voltage, and luminous efficiency are values at a driving current of 20 mA/cm², and they exhibit initial characteristics. LT70 is a time period needed for the initial luminance to be reduced to 70% thereof, and it represents lifetime characteristics.

**[Table 1]**

| | Host compound | Voltage (V) | Luminance (cd/m²) | Power efficiency (Im/W) | LT70 (h) |
|---|---|---|---|---|---|
| Example 1 | 128 | 4.50 | 10400 | 36.6 | 280 |
| Example 2 | 155 | 4.50 | 10500 | 36.6 | 290 |
| Example 3 | 112 | 4.30 | 10000 | 36.5 | 350 |
| Example 4 | 120 | 4.20 | 10100 | 37.8 | 340 |
| Example 5 | 122 | 4.40 | 10100 | 36.8 | 350 |
| Example 6 | 129 | 4.20 | 10100 | 37.8 | 330 |
| Example 7 | 156 | 4.20 | 9700 | 37.0 | 320 |
| Comp. Example 1 | A | 4.60 | 10000 | 34.1 | 250 |
| Comp. Example 2 | B | 4.80 | 10300 | 33.7 | 130 |
| Comp. Example 3 | C | 4.65 | 9900 | 33.4 | 280 |
| Comp. Example 4 | D | 6.45 | 10700 | 26.0 | 50 |

### Example 8

On a glass substrate on which an anode made of ITO with a film thickness of 110 nm was formed, respective thin films were laminated by a vacuum evaporation method at a degree of vacuum of 4.0 × 10⁻⁵ Pa. First, HAT-CN was formed with a thickness of 25 nm as a hole injection layer on ITO, and next, NPD was formed with a thickness of 30 nm as a hole transport layer. Next, HT-1 was formed with a thickness of 10 nm as an electron blocking layer. Then, compound 128 as a first host, compound 602 as a second host and Ir(ppy)₃ as a light-emitting dopant were co-vapor-deposited from different vapor deposition sources, respectively, to form a light-emitting layer with a thickness of 40 nm. In this case, co-vapor deposition was performed under vapor deposition conditions such that the concentration of Ir(ppy)₃ was 10 wt%, and the weight ratio between the first host and the second host was 30:70. Next, ET-1 was formed with a thickness of 20 nm as an electron transport layer. Further, LiF was formed with a thickness of 1 nm as an electron injection layer on the electron transport layer. Finally, Al was formed with a thickness of 70 nm as a cathode on the electron injection layer to produce an organic EL device.

### Examples 9 to 14, 22 to 48

Organic EL devices were produced in the same manner as in Example 8 except that compounds shown in Table 2 were used as the first host and the second host instead of those of Example 8.

### Examples 15 to 21

Organic EL devices were produced in the same manner as in Example 8 except that compounds shown in Table 2 were used as the first host and the second host instead of those of Example 8, and co-vapor deposition was performed in Example 8 under vapor deposition conditions such that the weight ratio between the first host and the second host was 40:60.

### Examples 49 to 51

Organic EL devices were produced in the same manner as in Example 8 except that a premixture obtained by weighing a first host (0.30 g) and a second host (0.70 g) and mixing them while grinding in a mortar was co-vapor-deposited from one vapor deposition source.

### Comparative Examples 6 to 10, 16 to 18

Organic EL devices were produced in the same manner as in Example 8 except that compounds shown in Table 2 were used as the first host and the second host instead of those of Example 8.

### Comparative Examples 11 to 15

Organic EL devices were produced in the same manner as in Example 15 except that compounds shown in Table 2 were used as the first host and the second host instead of those of Example 15.

Evaluation results of the produced organic EL devices are shown in Tables 2 and 3. In the tables, the luminance, driving voltage, and luminous efficiency, and LT70 are the same as those in Table 1.

**[Table 2]**

| | First host compound | Second host compound | Voltage (V) | Luminance (cd/m²) | Power efficiency (Im/W) | LT70 (h) |
|---|---|---|---|---|---|---|
| Example 8 | 128 | 602 | 4.30 | 11300 | 41.3 | 2700 |
| Example 9 | 155 | 602 | 4.30 | 10900 | 39.8 | 2760 |
| Example 10 | 112 | 602 | 4.10 | 10700 | 41.0 | 3100 |
| Example 11 | 120 | 602 | 4.20 | 10900 | 40.7 | 3150 |
| Example 12 | 122 | 602 | 4.10 | 11500 | 44.0 | 3170 |
| Example 13 | 129 | 602 | 4.30 | 11300 | 41.3 | 3130 |
| Example 14 | 156 | 602 | 4.30 | 10900 | 39.8 | 2910 |
| Example 15 | 128 | 602 | 4.00 | 11700 | 45.9 | 2600 |
| Example 16 | 155 | 602 | 4.10 | 11100 | 42.5 | 2500 |
| Example 17 | 112 | 602 | 3.80 | 10700 | 44.2 | 3000 |
| Example 18 | 120 | 602 | 3.90 | 11100 | 44.7 | 3000 |
| Example 19 | 122 | 602 | 3.80 | 12200 | 50.4 | 2900 |
| Example 20 | 129 | 602 | 4.10 | 11600 | 44.4 | 2900 |
| Example 21 | 156 | 602 | 3.90 | 11300 | 45.5 | 2700 |
| Example 22 | 128 | 640 | 4.20 | 10700 | 40.0 | 2990 |
| Example 23 | 155 | 640 | 4.20 | 11800 | 44.1 | 2850 |
| Example 24 | 112 | 640 | 4.00 | 11100 | 43.6 | 3320 |
| Example 25 | 120 | 640 | 4.10 | 10500 | 40.2 | 3420 |
| Example 26 | 122 | 640 | 4.10 | 11200 | 42.9 | 3390 |
| Example 27 | 129 | 640 | 4.10 | 11000 | 42.1 | 3640 |
| Example 28 | 156 | 640 | 4.20 | 11100 | 41.5 | 3220 |
| Example 29 | 128 | 818 | 4.20 | 11100 | 41.5 | 1610 |
| Example 30 | 155 | 818 | 4.20 | 10700 | 40.0 | 1590 |
| Example 31 | 112 | 818 | 4.00 | 10800 | 42.4 | 2030 |
| Example 32 | 120 | 818 | 3.90 | 10600 | 42.7 | 1960 |
| Example 33 | 122 | 818 | 4.00 | 11100 | 43.6 | 2100 |
| Example 34 | 129 | 818 | 4.10 | 11100 | 42.5 | 1970 |
| Example 35 | 156 | 818 | 4.10 | 10800 | 41.4 | 1850 |
| Example 36 | 128 | 941 | 4.20 | 11300 | 42.2 | 1580 |
| Example 37 | 155 | 941 | 4.00 | 11200 | 44.0 | 1520 |

**[Table 3]**

| | First host compound | Second host compound | Voltage (V) | Luminance (cd/m2) | Power efficiency (Im/W) | LT70 (h) |
|---|---|---|---|---|---|---|
| Example 38 | 112 | 941 | 3.90 | 11100 | 44.7 | 1820 |
| Example 39 | 120 | 941 | 3.90 | 11100 | 44.7 | 1710 |
| Example 40 | 122 | 941 | 4.00 | 11000 | 43.2 | 1850 |
| Example 41 | 129 | 941 | 4.10 | 11900 | 45.6 | 1920 |
| Example 42 | 156 | 941 | 4.10 | 11500 | 44.0 | 1720 |
| Example 43 | 128 | 864 | 4.20 | 11700 | 43.7 | 1650 |
| Example 44 | 155 | 864 | 4.00 | 11500 | 45.1 | 1590 |
| Example 45 | 112 | 864 | 3.80 | 12300 | 50.8 | 1930 |
| Example 46 | 120 | 864 | 4.00 | 11800 | 46.3 | 1940 |
| Example 47 | 122 | 864 | 4.00 | 12000 | 47.1 | 2000 |
| Example 48 | 129 | 864 | 4.00 | 11800 | 46.3 | 1940 |
| Example 49 | 112 | 640 | 4.00 | 11100 | 43.6 | 3350 |
| Example 50 | 120 | 640 | 4.10 | 10600 | 40.6 | 3580 |
| Example 51 | 129 | 602 | 4.30 | 11500 | 42.0 | 3200 |
| Comp. Example 6 | A | 602 | 4.40 | 10800 | 38.5 | 2400 |
| Comp. Example 7 | B | 602 | 4.65 | 11100 | 37.5 | 1300 |
| Comp. Example 8 | C | 602 | 4.45 | 10400 | 36.7 | 2600 |
| Comp. Example 9 | D | 602 | 6.15 | 11700 | 29.9 | 600 |
| Comp. Example 10 | E | 602 | 4.45 | 11000 | 38.8 | 2500 |
| Comp. Example 11 | A | 602 | 4.05 | 11000 | 42.6 | 2200 |
| Comp. Example 12 | B | 602 | 4.35 | 11400 | 41.1 | 1200 |
| Comp. Example 13 | C | 602 | 4.10 | 10500 | 40.2 | 2400 |
| Comp. Example 14 | D | 602 | 5.90 | 12100 | 32.2 | 500 |
| Comp. Example 15 | E | 602 | 4.20 | 11400 | 42.6 | 2300 |
| Comp. Example 16 | A | 640 | 4.30 | 10800 | 39.4 | 2500 |
| Comp. Example 17 | A | 818 | 4.25 | 10700 | 39.5 | 1500 |
| Comp. Example 18 | A | 941 | 4.25 | 11100 | 41.0 | 1400 |
| Comp. Example 19 | A | 864 | 4.25 | 11100 | 41.0 | 1400 |

Compounds used in Examples are shown below.

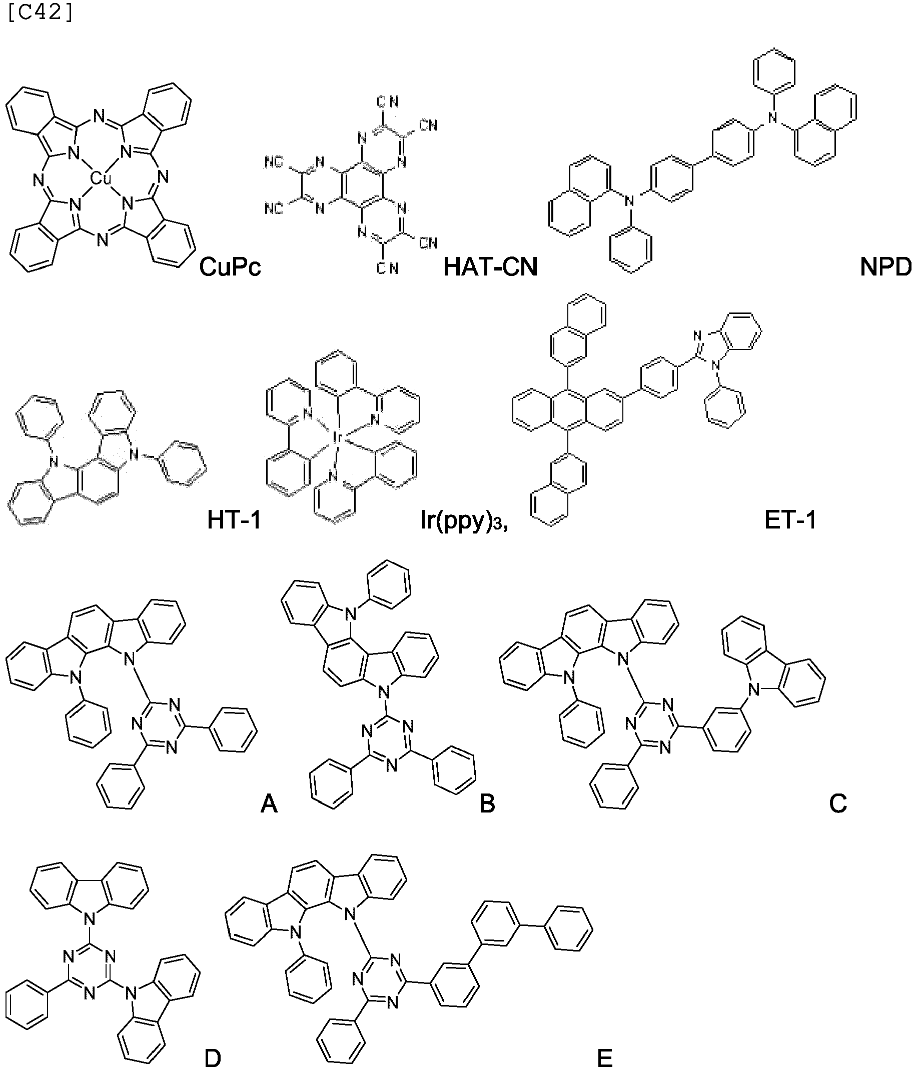

Table 4 shows the 50% weight reduction temperatures (T₅₀) of compounds 112, 120, 129, 602, and 640.

**[Table 4]**

| Compound | T₅₀[°C] |
|---|---|
| 112 | 335 |
| 120 | 324 |
| 129 | 280 |
| 602 | 279 |
| 640 | 317 |

## Claims

1. A material for an organic electroluminescent device comprising a compound represented by the following general formula (1): 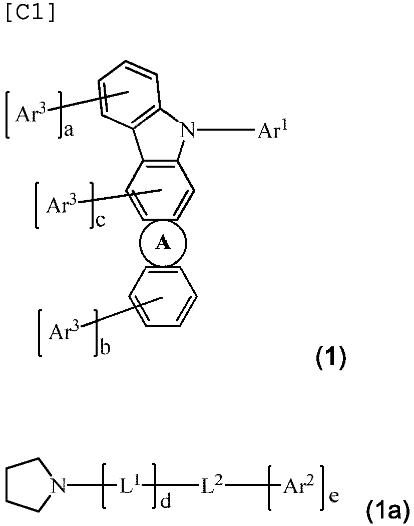
wherein a ring A is a heterocycle represented by formula (1a), and the ring A is fused to an adjacent ring at any position;
Ar¹ represents a substituted or unsubstituted aromatic hydrocarbon group having 6 to 30 carbon atoms, a substituted or unsubstituted aromatic heterocyclic group having 3 to 11 carbon atoms, or a substituted or unsubstituted linked aromatic group in which two to five of these aromatic rings are linked to each other;
Ar² independently represents a substituted or unsubstituted aromatic hydrocarbon group having 6 to 30 carbon atoms, a substituted or unsubstituted aromatic heterocyclic group having 3 to 17 carbon atoms, or a substituted or unsubstituted linked aromatic group in which two to five of these aromatic rings are linked to each other;
L¹ independently represents a substituted or unsubstituted aromatic hydrocarbon group having 6 to 30 carbon atoms, or a substituted or unsubstituted aromatic heterocyclic group having 3 to 11 carbon atoms;
L² represents a substituted or unsubstituted aromatic heterocyclic group having 3 to 11 carbon atoms;
Ar³ independently represents a substituted or unsubstituted aromatic hydrocarbon group having 6 to 30 carbon atoms, or a substituted or unsubstituted linked aromatic group in which two to five of the aromatic hydrocarbon groups are linked to each other; and
a and b each independently represent an integer of 0 to 4, and c represents an integer of 0 to 2, provided that a + b + c ≥ 1.
d represents the number of repetitions and an integer of 0 to 3, and e represents the number of substitutions and an integer of 0 to 5.

2. The material for an organic electroluminescent device according to claim 1, wherein L² represents a substituted or unsubstituted aromatic heterocyclic group having 3 to 5 carbon atoms.

3. The material for an organic electroluminescent device according to claim 1 or 2, wherein Ar¹, Ar², and L¹ each independently represent a substituted or unsubstituted aromatic hydrocarbon group having 6 to 24 carbon atoms, or a substituted or unsubstituted linked aromatic group in which two to five of the aromatic hydrocarbon groups are linked to each other.

4. The material for an organic electroluminescent device according to any one of claims 1 to 3, wherein d represents 0.

5. The material for an organic electroluminescent device according to claim 4, wherein the compound represented by the general formula (1) is a compound represented by any of the following general formulas (2) to (7): 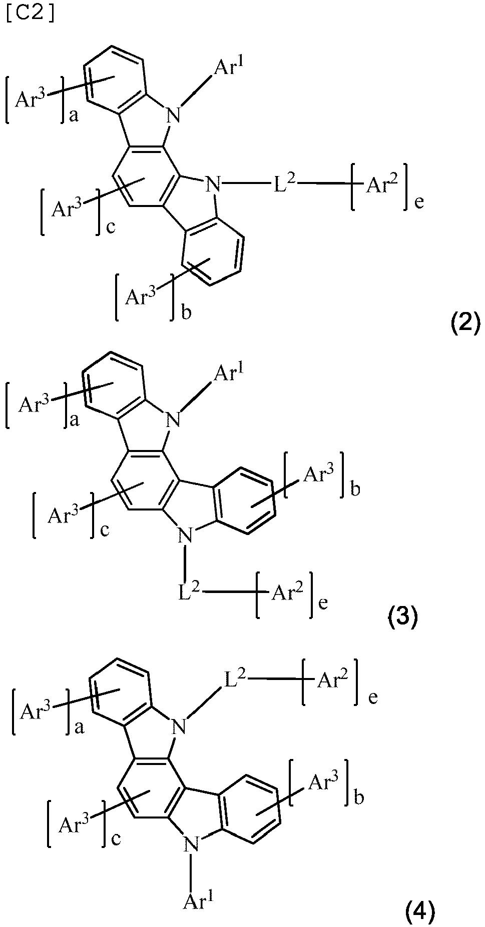 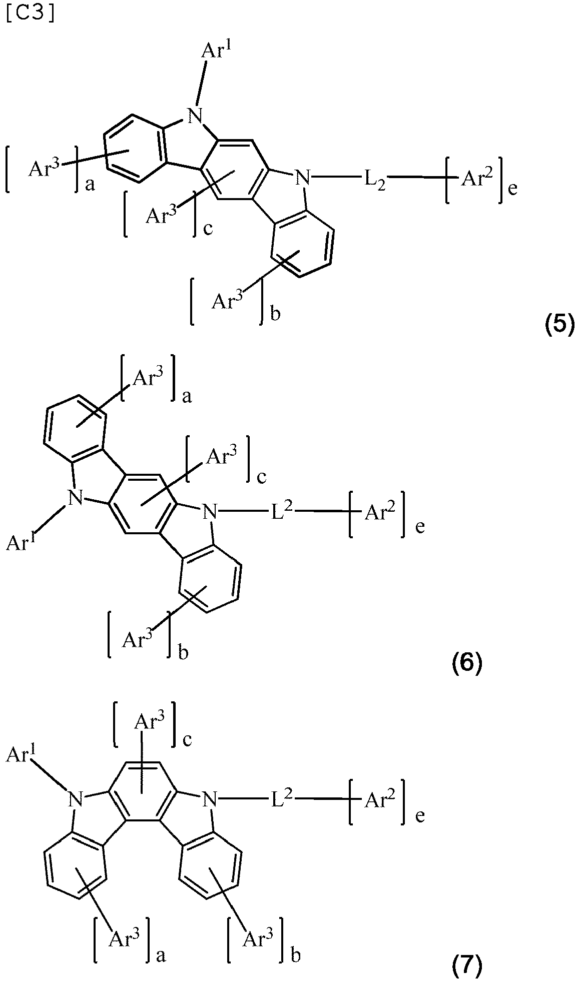 wherein L², Ar¹, Ar², Ar³, a, b, c, and e are as defined for the general formula (1).

6. The material for an organic electroluminescent device according to any one of claims 1 to 5, wherein at least one selected from a and b represents 0.

7. The material for an organic electroluminescent device according to any one of claims 1 to 6, wherein c is 1 or 2.

8. An organic electroluminescent device having a plurality of organic layers between an anode and a cathode, wherein at least one layer of the organic layers comprises the material for an organic electroluminescent device according to any one of claims 1 to 7.

9. The organic electroluminescent device according to claim 8, comprising the material for an organic electroluminescent device and also at least one of compounds represented by the following general formulas (8) to (10) in the same layer: 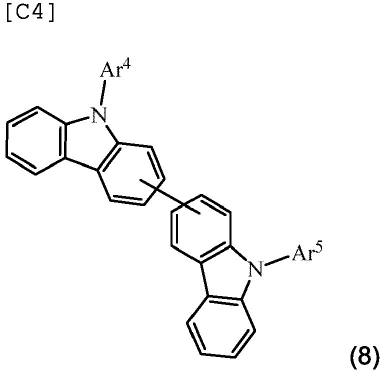
wherein Ar⁴ and Ar⁵ each independently represent a substituted or unsubstituted aromatic hydrocarbon group having 6 to 30 carbon atoms, a substituted or unsubstituted aromatic heterocyclic group having 3 to 17 carbon atoms, or a substituted or unsubstituted linked aromatic group in which two to five of these aromatic rings are linked to each other; 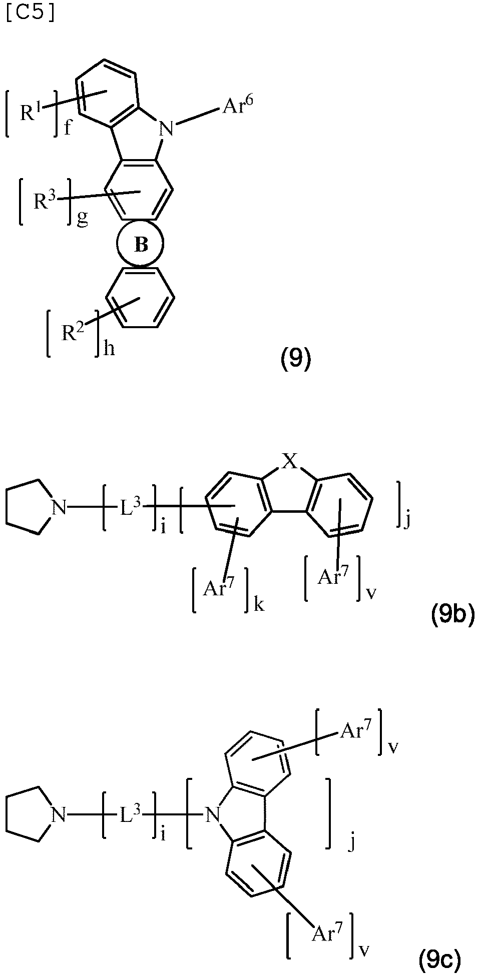
wherein a ring B is a heterocycle represented by formula (9b) or (9c), and the ring B is fused to an adjacent ring at any position;
L³ independently represents a substituted or unsubstituted aromatic hydrocarbon group having 6 to 30 carbon atoms;
X represents NAr⁸, O, or S;
Ar⁶, Ar⁷, and Ar⁸ each independently represent a substituted or unsubstituted aromatic hydrocarbon group having 6 to 30 carbon atoms, a substituted or unsubstituted aromatic heterocyclic group having 3 to 17 carbon atoms, or a substituted or unsubstituted linked aromatic group in which two to five of these aromatic rings are linked to each other; and
i and j each independently represent an integer of 0 to 3, k and v represent the number of substitutions, k represents an integer of 0 to 3, and v independently represents an integer of 0 to 4, provided that i + j is an integer of 1 or more;
R¹ to R³ each independently represent a cyano group, an alkyl group having 1 to 20 carbon atoms, an aralkyl group having 7 to 38 carbon atoms, an alkenyl group having 2 to 20 carbon atoms, an alkynyl group having 2 to 20 carbon atoms, a dialkylamino group having 2 to 40 carbon atoms, a diarylamino group having 12 to 44 carbon atoms, a diaralkylamino group having 14 to 76 carbon atoms, an acyl group having 2 to 20 carbon atoms, an acyloxy group having 2 to 20 carbon atoms, an alkoxy group having 1 to 20 carbon atoms, an alkoxycarbonyl group having 2 to 20 carbon atoms, an alkoxycarbonyloxy group having 2 to 20 carbon atoms, an alkylsulfonyl group having 1 to 20 carbon atoms, a substituted or unsubstituted aromatic hydrocarbon group having 6 to 30 carbon atoms, or a substituted or unsubstituted aromatic heterocyclic group having 3 to 17 carbon atoms; and
f and h each independently represent an integer of 0 to 4, and g represents an integer of 0 to 2; 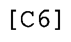 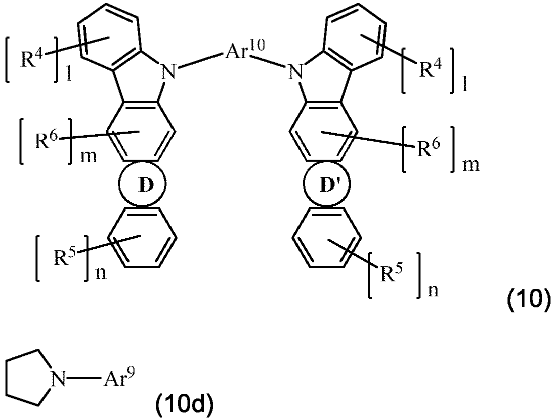
wherein rings D and D' are each a heterocycle represented by formula (10d), and the rings D and D' are each independently fused to an adjacent ring at any position;
Ar⁹ independently has the same meaning as Ar⁶ in the general formula (9);
R⁴ to R⁶ each independently have the same meaning as R¹ to R³ in the general formula (9); and
l and n each independently represent an integer of 0 to 4, and m independently represents an integer of 0 to 2; and
Ar¹⁰ represents a substituted or unsubstituted aromatic hydrocarbon group having 6 to 30 carbon atoms, a substituted or unsubstituted aromatic heterocyclic group having 3 to 17 carbon atoms, or a substituted or unsubstituted linked aromatic group in which two to five of these aromatic rings are linked to each other.

10. The organic electroluminescent device according to claim 8 or 9, wherein the organic layer comprising the material for an organic electroluminescent device is at least one layer selected from the group comprising of a light-emitting layer, a hole injection layer, a hole transport layer, an electron transport layer, an electron injection layer, a hole blocking layer, and an electron blocking layer.

11. The organic electroluminescent device according to claim 10, wherein the organic layer comprising the material for an organic electroluminescent device is a light-emitting layer, and the light-emitting layer contains at least one light-emitting dopant.

12. A method for producing the organic electroluminescent device according to claim 9 or 10, comprising providing a mixed composition comprising the material for an organic electroluminescent device and at least one of the compounds represented by the general formula (8) to (10), and producing a light-emitting layer by use of the mixed composition.

13. A mixed composition used in the method for producing the organic electroluminescent device according to claim 12, comprising the material for an organic electroluminescent device and at least one of the compounds represented by the general formulas (8) to (10).

14. The mixed composition according to claim 13, wherein a difference in 50% weight reduction temperatures of the material for an organic electroluminescent device and the compound represented by the general formula (8), the compound represented by the general formula (9), or the compound represented by the general formula (10) is within 20°C.

15. The mixed composition according to claim 13 or 14, comprising the material for an organic electroluminescent device and the compound represented by the general formula (8).
